# EUROPEAN PATENT SPECIFICATION

(11) **EP 4 213 958 B1**
(45) Date of publication and mention of the grant of the patent: **18.02.2026**
(21) Application number: 21789988.9
(22) Date of filing: 16.09.2021
(51) Int. Cl.: B01D 15/16, B01D 15/20, B01D 15/34, B01J 20/28, B01J 20/32, B01J 20/286, G01N 30/34

(54) **IMPROVED SIZE EXCLUSION CHROMATOGRAPHY UTILIZING LOW CONCENTRATION AMINO ACIDS IN SIZE EXCLUSION CHROMATOGRAPHY MOBILE PHASE**
VERBESSERTE GRÖSSENAUSSCHLUSSCHROMATOGRAFIE UNTER VERWENDUNG VON NIEDRIGKONZENTRIERTEN AMINOSÄUREN IN DER MOBILEN PHASE DER GRÖSSENAUSSCHLUSSCHROMATOGRAFIE
CHROMATOGRAPHIE D'EXCLUSION STÉRIQUE AMÉLIORÉE UTILISANT DES ACIDES AMINÉS À FAIBLE CONCENTRATION DANS UNE PHASE MOBILE DE CHROMATOGRAPHIE D'EXCLUSION STÉRIQUE

(30) Priority: 16.09.2020 US 202063079303 P
(43) Date of publication of application: 26.07.2023
(73) Proprietor: Waters Technologies Corporation, Milford, MA 01757 (US)
(72) Inventor: SCHMUDLACH, Andrew Wyatt, Ashland, Massachusetts 01721 (US); RZEWUSKI, Susan, Cumberland, Rhode Island 02864 (US); LAUBER, Matthew A., Smithfield, Rhode Island 02896 (US); TUNC SARISOZEN, Yeliz, Westford, Massachusetts 01886 (US); LAWRENCE, Nicole L., Springs, Connecticut 06076 (US); BROUSMICHE, Darryl W., Grafton, Massachusetts 01519 (US)
(74) Representative: Forresters IP LLP
(86) International application number: PCT/US2021/050743
(87) International publication number: WO 2022/061035

(56) References cited:
- WO-A1-2019/239329
- WO-A2-2011/084506
- WO-A2-2018/018011
- EJIMA D ET AL: "Arginine as an effective additive in gel permeation chromatography", JOURNAL OF CHROMATOGRAPHY A, ELSEVIER, AMSTERDAM, NL, vol. 1094, no. 1-2, 11 November 2005 (2005-11-11), pages 49 - 55, XP027722902, ISSN: 0021-9673, [retrieved on 20051111]
- KEVIN WYNDHAM: "A Review of Waters Hybrid Particle Technology. Part 2. Ethylene-Bridged [BEH Technology ™ ] Hybrids and Their Use in Liquid Chromatography", 29 September 2006 (2006-09-29), XP055689330, Retrieved from the Internet <URL:http://www.waters.com/webassets/cms/library/docs/720001159en.pdf> [retrieved on 20200424]

## Description

### TECHNICAL FIELD

The present disclosure relates to methods for performing size exclusion chromatography. Particularly, the disclosure relates to methods for improving separations of proteinaceous analytes in size exclusion chromatography, for example by using low concentrations of amino acids in the mobile phase.

### BACKGROUND

Size exclusion chromatography (SEC) is a common separation technique that employs differences in hydrodynamic radii to separate solubilized analytes. In theory, perfect SEC separates exclusively based on the hydrodynamic radii; however, secondary interactions, such as ionic and hydrophobic interactions, can cause undesired effects including peak broadening, tailing, and loss of resolution and separation efficiency. For separations of biopharmaceutical materials, such as monoclonal antibodies, antibody drug conjugates, or fusion proteins, these secondary interactions result in a significant analytical challenge. Traditional approaches to reduce these secondary interactions include the addition of salts, such as sodium or potassium chloride, or the inclusion of organic co-solvents, such as methanol, ethanol, isopropanol, or acetonitrile. However, there is no universal solution for all target analytes, and each desired separation requires optimization of mobile phase components. Mobile phase optimization is generally tedious, time consuming, and lacks ease of use for novice users.

The inclusion of moderate to high levels of a salt, while potentially of benefit in reducing secondary interactions, can necessitate a desalting step in the purification process, or preclude the use certain types of detectors, such as mass spectrometers (MS). The use of organic co-solvents presents a burden to the separation of native proteins; there is the omnipresent threat that the protein will irreversibly denature or adopt a conformation that diminishes or eliminates the value of the analyte.
WO 2011/084506 discloses a device and method for performing size exclusion chromatography.

### SUMMARY

The present disclosure is generally directed to methods for performing size exclusion chromatography (SEC). Methods are disclosed herein for performing SEC, for example, to separate, resolve, and/or analyze biomolecules. In general, methods of the present disclosure provide SEC separations with reduced secondary interactions while exhibiting compatibility with a ride range of analytes and the ability to use standard detection methods. The methods utilize a mobile phase supplemented with low concentrations of amino acids, including but not limited to modified amino acids, to help stabilize target analytes and improve chromatographic performance. Such methods are particularly suited for use with stationary phase materials bonded or coated with a polyethylene oxide (PEO), also referred to as polyethylene glycol (PEG). Surprisingly, according to the present disclosure, it has been found that adding low concentrations of certain amino acids or derivatives thereof to buffered mobile phases improved peak characteristics during SEC on PEO modified SEC particles. Advantageously, supplementation with low concentrations of certain amino acids or derivatives thereof do not diminish sensitivity for standard optical and mass spectrometry (MS) methods of detection. Further, in some embodiments, the chromatographic improvements are maintained with various buffers, pHs, and column temperatures.

In one aspect is provided a method for performing size exclusion chromatography on a sample containing at least one analyte, the method comprising:
a. contacting said sample with a column chromatography device comprising a column having an interior for accepting a stationary phase, and an immobilized stationary phase within said interior of the column, wherein the immobilized stationary phase comprises porous particles having a diameter with a mean size distribution of between about 1 and about 20 µm; an average pore size from about 40 to about 3000 Å; and wherein said porous particles are surface modified with a hydroxy-terminated polyethylene glycol at a surface concentration from about 0.5 to about 5.0 µmoles/m²;
b. flowing a mobile phase through the immobilized stationary phase for a period of time, the mobile phase comprising water; a buffer; and an amino acid or derivative thereof, wherein the amino acid or derivative thereof is present in the mobile phase at a concentration from about 5 to about 40 mM; and
c. eluting the at least one analyte from the immobilized stationary phase in the mobile phase.

In some embodiments, eluting comprises separating the sample into one or more analytes on the basis of decreasing hydrodynamic radius of said one or more analytes.

In some embodiments, the amino acid or derivative thereof is present in the mobile phase at a concentration from about 5 to about 20 mM. In some embodiments, the amino acid or derivative thereof is present in the mobile phase at a concentration of about 10 mM. In some embodiments, the amino acid is selected from the group consisting of L-arginine, L-ornithine, and L-lysine. In some embodiments, the amino acid derivative is an alkyl ester of the amino acid or an N-acylated amino acid. In some embodiments, the amino acid derivative is L-arginine methyl ester.

In some embodiments, the at least one analyte comprises a nucleic acid, a polysaccharide, a peptide, a polypeptide, or a protein. In some embodiments, the at least one analyte comprises an antibody. In some embodiments, the at least one analyte is an antibody-drug conjugate. In some embodiments, the at least one analyte comprises an adenovirus, an adeno-associated virus (AAV), mRNA, DNA, plasmids, exosomes, extracellular vesicles, lipid nanoparticle encapsulated nucleic acids, or combinations thereof. In some embodiments, the at least one analyte comprises an adenovirus. In some embodiments, the at least one analyte comprises an AAV.

In some embodiments, the method further comprises detecting the presence or absence of the at least one analyte in the sample. In some embodiments, the detecting is performed using a refractive index detector, a UV detector, a light-scattering detector, a mass spectrometer, or combinations thereof. In some embodiments, the detecting is performed using a UV detector.

In some embodiments, flowing the mobile phase through the immobilized stationary phase is performed at a flow rate from about 0.2 mL/min to about 3 mL/min.

In some embodiments, the period of time is less than 60 minutes, less than 50 minutes, less than 40 minutes, less than 30 minutes, less than 20 minutes, less than 10 minutes, less than 5 minutes, less than 4 minutes, less than 3 minutes, less than 2 minutes, or less than 1 minute.

In some embodiments, the buffer is present at a concentration from about 10 to about 100 mM. In some embodiments, the buffer is an alkali metal phosphate. In some embodiments, the buffer is sodium phosphate monobasic, sodium phosphate dibasic, or a combination thereof.

In some embodiments, a pH value of the mobile phase is from about 6.0 to about 7.5

In some embodiments, a column temperature is from about 20 to about 50°C.

In some embodiments, the mobile phase does not include an organic co-solvent, does not include a salt, or does not include either.

In some embodiments, the porous particles comprise silica, an inorganic-organic hybrid material, or a polymer. In some embodiments, the porous particles comprise silica. In some embodiments, the porous particles comprise an inorganic-organic hybrid material. In some embodiments, the porous particles comprise inorganic-organic hybrid particles having an empirical formula of SiO₂(O_{1.5}SiCH₂CH₂SiO_{1.5})_{0.25}. In some embodiments, the porous particles comprise an inorganic-organic hybrid material. the porous hybrid material particles having an average pore size from about 40 to about 1000 Å, from about 100 to about 500 Å, or from about 100 to about 300 Å.

In some embodiments, the hydroxy-terminated polyethylene glycol has the formula 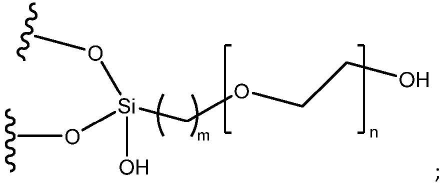 wherein:
m is an integer from about 1 to about 10;
n is an integer from about 2 to about 50; and
wherein the wavy lines indicate points of attachment to the surface of the porous particles.

In some embodiments, m is 2 or 3.

In some embodiments, n is from about 5 to about 15, or from about 8 to about 12.

In some embodiments, m is 3 and n is from about 8 to about 12.

In some embodiments, the porous particles comprise porous silica particles having a surface, at least some substantial portion thereof modified with a hydroxy-terminated polyethylene glycol, In some embodiments, the surface modified porous silica particles have an average pore size from about 250 to about 3000 Å, or from about 1000 to about 3000 Å, or from about 1000 to about 2000 Å, and at least a portion of the surface is modified with a methoxy-terminated polyethylene glycol.In some embodiments, the portion of the surface modified with the methoxy-terminated polyethylene glycol is the result of treatment of the stationary phase material with a methoxy-terminated polyethylene glycol reagent having a formula: 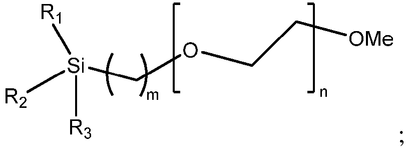 wherein:
at least one of R₁, R₂, and R₃ is OMe, OEt, Cl, or N(CH₃)₂;
m is an integer from about 1 to about 10; and
n is an integer from about 3 to about 20.

In some embodiments, m is 1, 2, 3, 4, 5, 6, 7, 8, 9, or 10. In some embodiments, m is 2 or 3. In some embodiments, m is 3 (i.e., propyl).

In some embodiments, n is from about 5 to about 15. In some embodiments, n is from about 6 to about 12, such as from about 6 to about 9.

In some embodiments, the methoxy-terminated PEG reagent is 2-[methoxy(polyethyleneoxy)₆₋₉propyl]trichlorosilane or 2-[methoxy(polyethyleneoxy)₆₋₉propyl]tris(dimethylamino)silane.

In some embodiments, a secondary interaction between the at least one analyte and the stationary phase are reduced relative to size exclusion chromatography performed using a mobile phase which does not comprise an amino acid or derivative thereof, the reduction of the secondary interaction characterized by an improvement in one or more of peak shape, peak area, peak tailing, analyte recovery, or decreased inter-run variability.

In another aspect is provided a method for reducing a secondary interaction in size exclusion chromatography, the method comprising:
a. providing a sample including at least one analyte;
b. providing a column chromatography device configured to detect the presence or absence of least one analyte in a sample, the column chromatography device comprising a column having an interior for accepting a stationary phase, and an immobilized stationary phase within said interior of the column, wherein the immobilized stationary phase comprises porous particles having a diameter with a mean size distribution of between about 1 and about 20 µm; an average pore size from about 40 to about 3000 Å; and wherein said porous particles are surface modified with a hydroxy-terminated polyethylene glycol at a surface concentration from about 0.5 to about 5.0 µmoles/m²;
c. providing a mobile phase comprising water; a buffer; and an amino acid or derivative thereof, wherein the amino acid or derivative thereof is present in the mobile phase at a concentration from about 5 to about 40 mM;
d. injecting the sample onto the immobilized stationary phase;
e. flowing the mobile phase through the immobilized stationary phase for a period of time;
f. eluting the at least one analyte from the immobilized stationary phase in the mobile phase; and
g. detecting the presence of the least one analyte in the sample, wherein a peak in a chromatogram indicates the presence of the least one analyte in the sample, and wherein the reduction of the secondary interaction is characterized by an improvement in one or more of peak shape, peak area, peak tailing, analyte recovery, or decreased inter-run variability.

### BRIEF DESCRIPTION OF THE DRAWINGS

In order to provide an understanding of embodiments of the technology, reference is made to the appended drawings, which are not necessarily drawn to scale. The drawings are exemplary only, and should not be construed as limiting the technology. The disclosure described herein is illustrated by way of example and not by way of limitation in the accompanying figures.

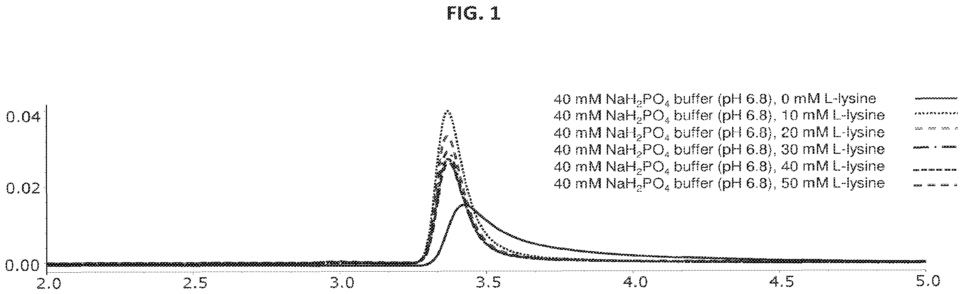

**FIG. 1** depicts exemplary chromatographic separations of Trastuzumab emtansine (Kadcyla; Genentech) on a prototype SEC column packed with 1.7 µm particles with average pore sizes of 270 Å having MeO-PEO(6-9 EO)propyltris(dimethylamino)silane bonding on a HO-PEO (8-12 EO)-TEOS coating , with a mobile phase comprising aqueous sodium phosphate buffer and varying concentrations of L-lysine.

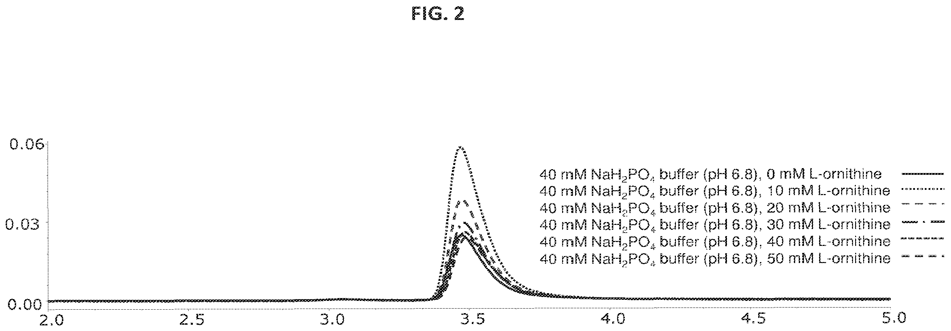

**FIG. 2** depicts exemplary chromatographic separations of Kadcyla on a prototype SEC column packed with 1.7 µm particles with average pore sizes of 270 Å having MeO-PEO(6-9 EO)propyltris(dimethylamino)silane bonding on a HO-PEO (8-12 EO)-TEOS coating, with a mobile phase comprising aqueous sodium phosphate buffer and varying concentrations of L-ornithine.

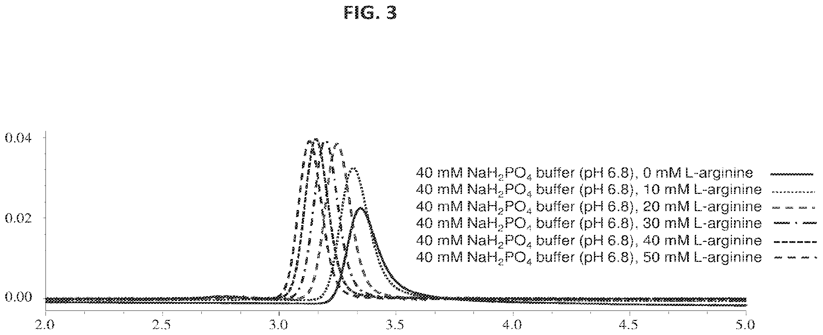

**FIG. 3** depicts exemplary chromatographic separations of Kadcyla on a prototype SEC column packed with 1.7 µm particles with average pore sizes of 270 Å having HO-PEO(8-12 EO)triethoxysilane bonding, with a mobile phase comprising aqueous sodium phosphate buffer and varying concentrations of L-arginine.

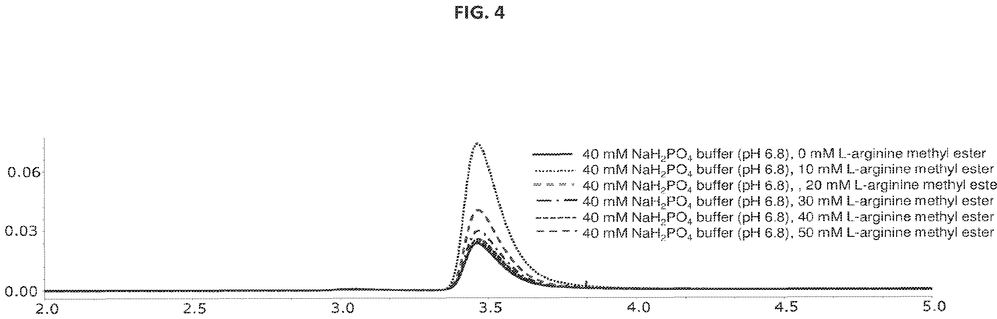

**FIG. 4** depicts exemplary chromatographic separations of Kadcyla on a prototype SEC column packed with 1.7 µm particles with average pore sizes of 270 Å having MeO-PEO(6-9 EO)propyltris(dimethylamino)silane bonding on a HO-PEO (8-12 EO)-TEOS coating, with a mobile phase comprising aqueous sodium phosphate buffer and varying concentrations of L-arginine methyl ester.

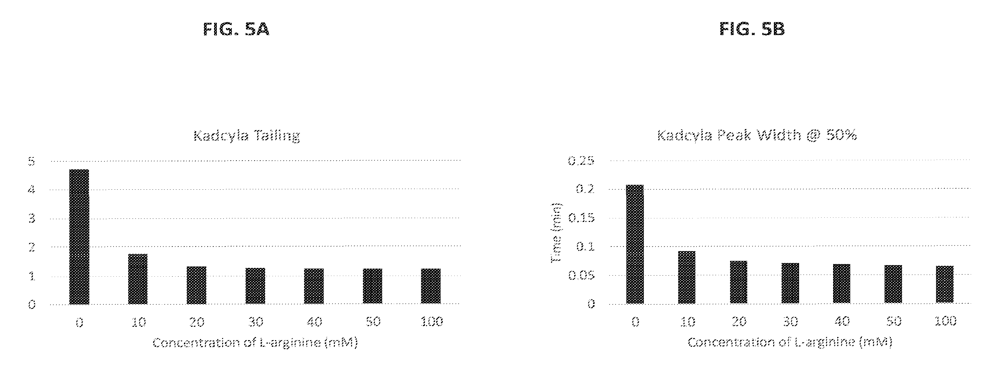

**FIG. 5A** depicts the peak tailing in an exemplary chromatographic separation of Kadcyla on a prototype polyethylene oxide (PEO) bonded SEC column packed with 1.7 µm particles with average pore sizes of 270 Å, with a mobile phase comprising aqueous sodium phosphate buffer and varying concentrations of L-arginine.
**FIG. 5B** depicts the peak width at half height in an exemplary chromatographic separation of Kadcyla on a prototype polyethylene oxide (PEO) bonded SEC column packed with 1.7 µm particles with average pore sizes of 270 Å, with a mobile phase comprising aqueous sodium phosphate buffer and varying concentrations of L-arginine.

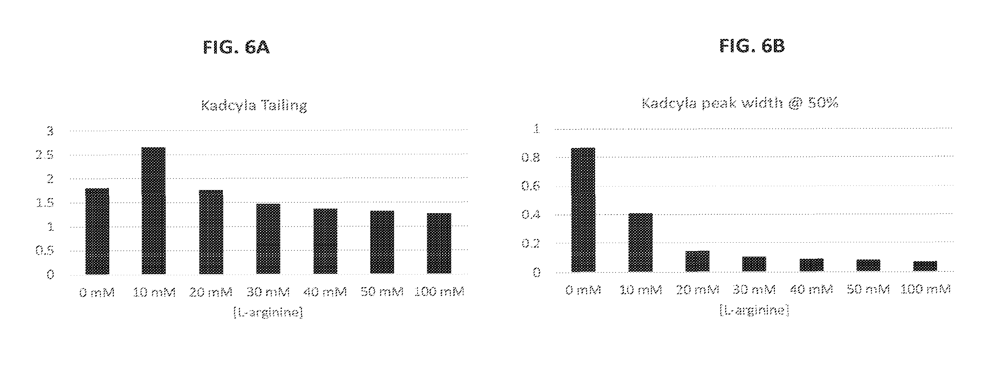

**FIG. 6A** depicts the peak tailing in an exemplary chromatographic separation of Kadcyla on a commercially available SEC column (BEH200; Waters Inc., pore size of 200 Å, 1.7 µm) with a mobile phase comprising aqueous sodium phosphate buffer and varying concentrations of L-arginine.
**FIG. 6B** depicts the peak width at half height in an exemplary chromatographic separation of Kadcyla on a commercially available SEC column (BEH200; Waters Inc., pore size of 200Å, 1.7 µm) with a mobile phase comprising aqueous sodium phosphate buffer and varying concentrations of L-arginine.

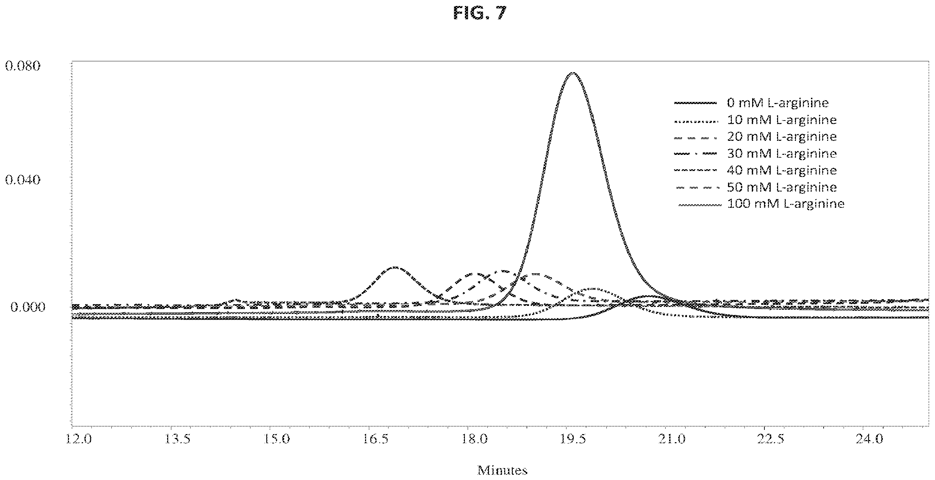

**FIG. 7** depicts exemplary chromatographic separations of Kadcyla on a commercially available SEC column (BioSuite; Waters Inc., pore size of 250 Å, 10 µm silica particles), with a mobile phase comprising aqueous sodium phosphate buffer and varying concentrations of L-arginine.

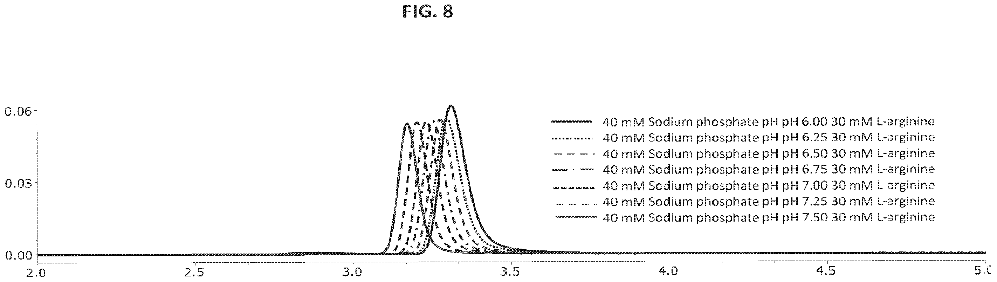

**FIG. 8** depicts exemplary chromatographic separations of Kadcyla on a prototype SEC column packed with 1.7 µm particles with average pore sizes of 270 Å having HO-PEO(8-12 EO)triethoxysilane bonding, with a mobile phase comprising 30 mM L-arginine and aqueous sodium phosphate buffer at various pH values.

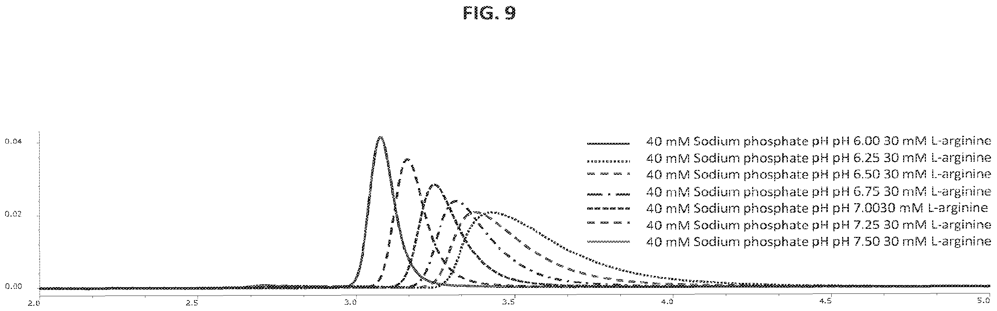

**FIG. 9** depicts exemplary chromatographic separations of Kadcyla on a commercially available SEC column (BEH200; Waters Inc., pore size of 200 Å, 1.7 µm), with a mobile phase comprising 30 mM L-arginine and aqueous sodium phosphate buffer at various pH values.

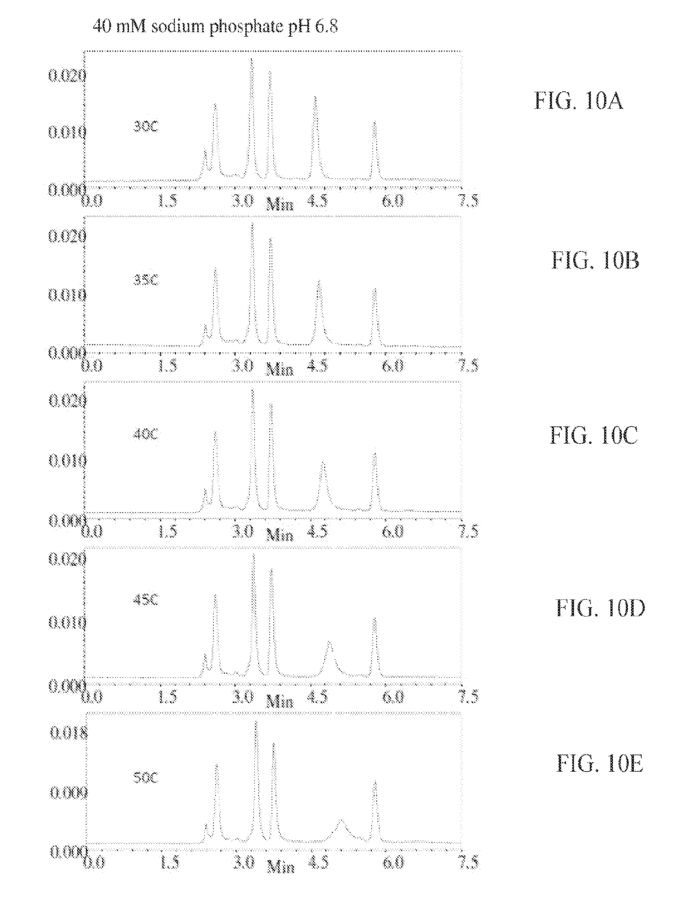

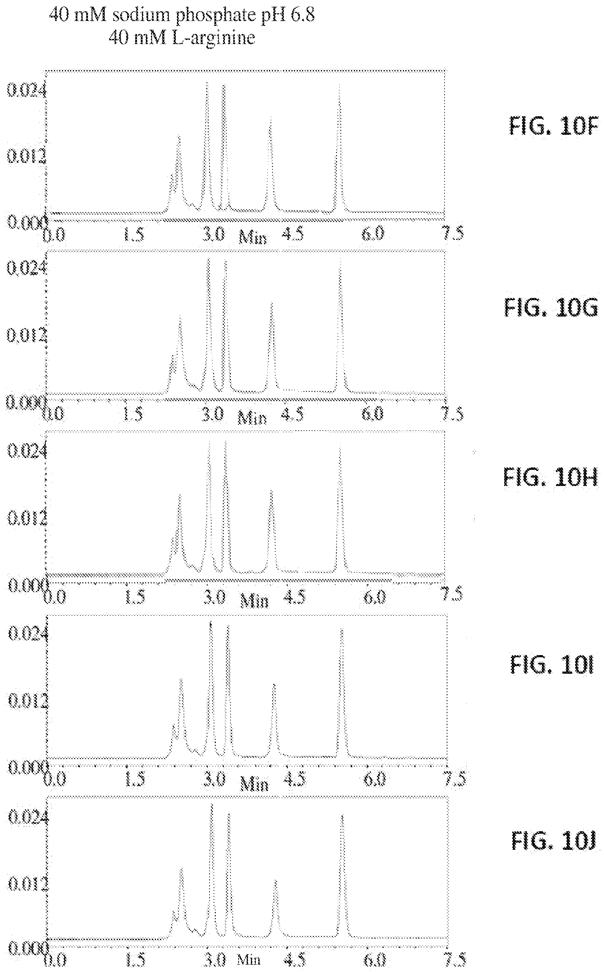

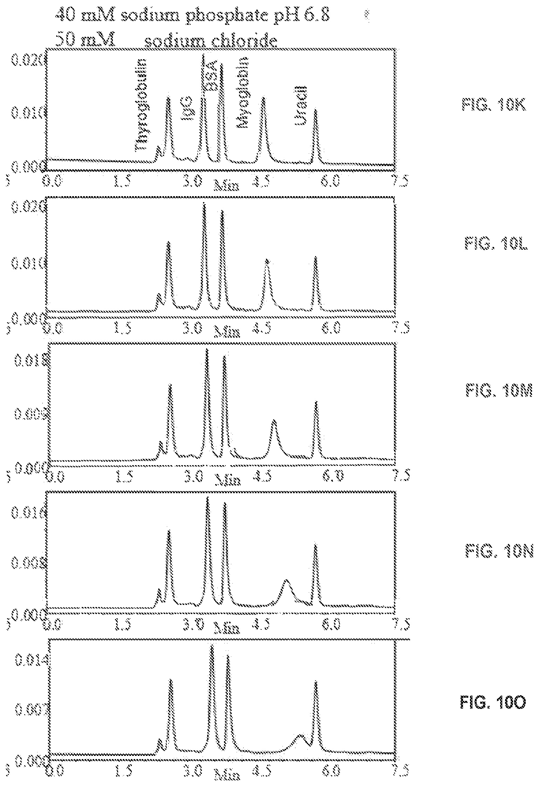

**FIGS. 10A-10O** depict exemplary chromatographic separations of a BEH200 protein mixture standard (Waters, Inc.) containing thyroglobulin, IgG, BSA, Myoglobin, and uracil, on a prototype SEC column packed with 1.7 µm particles with average pore sizes of 270 Å having HO-PEO(8-12 EO)triethoxysilane bonding, using three different mobile phases (40 mM sodium phosphate, 40 mM sodium phosphate with 40 mM L-arginine, and 40 mM sodium phosphate with 50 mM sodium chloride).at five different temperatures (30-50°C). **FIGS. 10A, 10B, 10C, 10D,** and **10E** depict the series of different temperature results for a mobile phase of 40 mM sodium phosphate. **FIGS. 10F, 10G. 10H, 10I,** and **10J** depict the series of different temperature results for a mobile phase of 40 mM sodium phosphate with 40 mM L-arginine. **FIGS. 10K, 10L, 10M, 10N,** and **10O** depict the series of different temperature results for a mobile phase of 40 mM sodium phosphate with 50 mM sodium chloride.

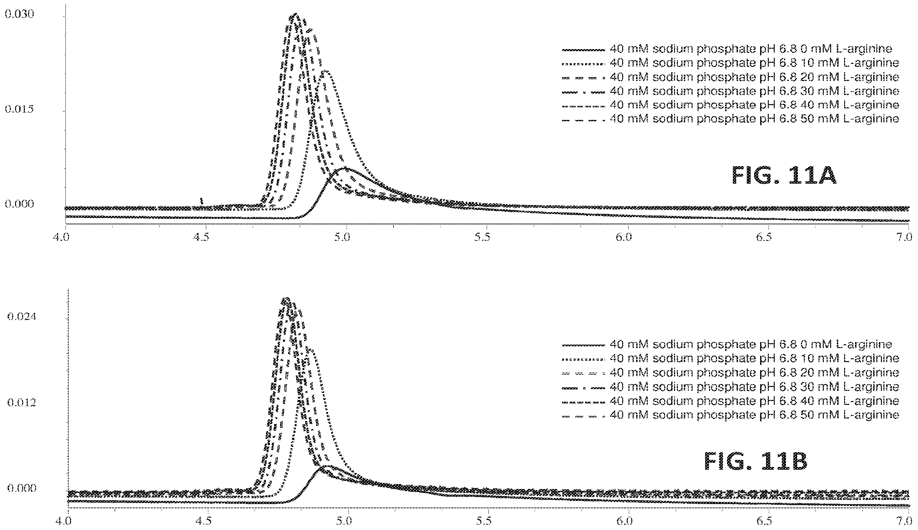

**FIG. 11A** depicts exemplary chromatographic separations of Kadcyla on a prototype SEC column packed with 3 µm HO-PEO(8-12 EO) triethoxysilane bonded silica particles with average pore sizes of 1000 Å, with a mobile phase comprising aqueous sodium phosphate buffer and varying concentrations of L-arginine.
**FIG. 11B** depicts exemplary chromatographic separations of Kadcyla on a prototype SEC column packed with 3 µm, 1000 Å silica particles with HO-PEO(8-12 EO) triethoxysilane bonding on a hybrid coating of (1,2-bis(triethoxysilyl)ethane (BTEE) and tetraethyl orthosilicate (TEOS), with a mobile phase comprising aqueous sodium phosphate buffer and varying concentrations of L-arginine.

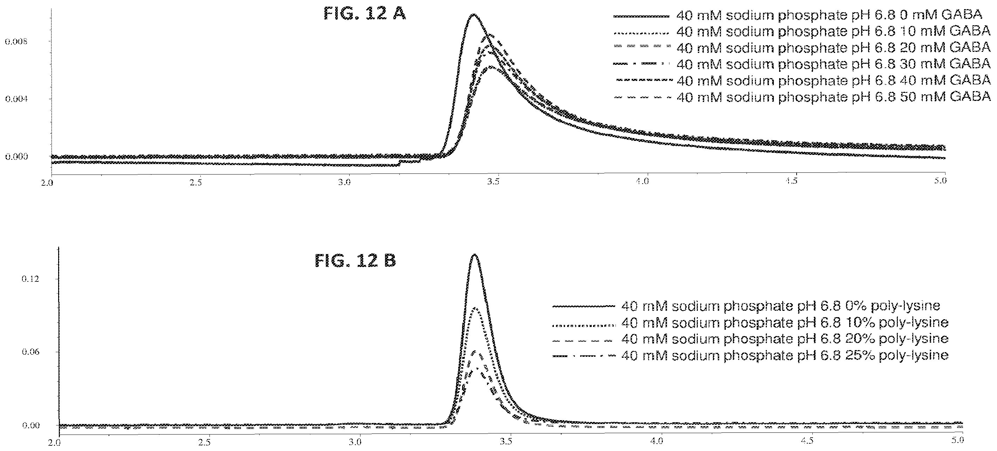

**FIG. 12A** depicts exemplary chromatographic separations of Kadcyla on a prototype SEC column packed with 1.7 µm particles with average pore sizes of 270 Å having MeO-PEO(6-9 EO)propyltris(dimethylamino)silane bonding on a HO-PEO (8-12 EO)-TEOS coating, with a mobile phase comprising aqueous sodium phosphate buffer and varying concentrations of gamma-aminobutyric acid.
**FIG. 12B** depicts exemplary chromatographic separations of Kadcyla on a prototype SEC column packed with 1.7 µm particles with average pore sizes of 270 Å having MeO-PEO(6-9 EO)propyltris(dimethylamino)silane bonding on HO-PEO (8-12 EO)-TEOS coating, with a mobile phase comprising aqueous sodium phosphate buffer and varying concentrations of poly-L-histidine.

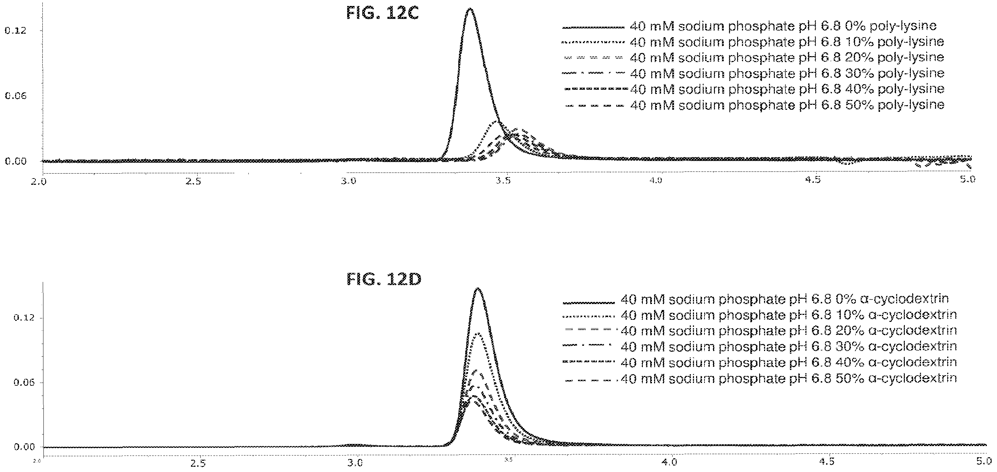

**FIG. 12C** depicts exemplary chromatographic separations of Kadcyla on a prototype SEC column packed with 1.7 µm particles with average pore sizes of 270 Å having MeO-PEO(6-9 EO)propyltris(dimethylamino)silane bonding on HO-PEO (8-12 EO)-TEOS coating, with a mobile phase comprising aqueous sodium phosphate buffer and varying concentrations of poly-L-lysine.
**FIG. 12D** depicts exemplary chromatographic separations of Kadcyla on a prototype SEC column packed with 1.7 µm particles with average pore sizes of 270 Å having MeO-PEO(6-9 EO)propyltris(dimethylamino)silane bonding on a HO-PEO (8-12 EO)-TEOS coating, with a mobile phase comprising aqueous sodium phosphate buffer and varying concentrations of α-cyclodextrin.

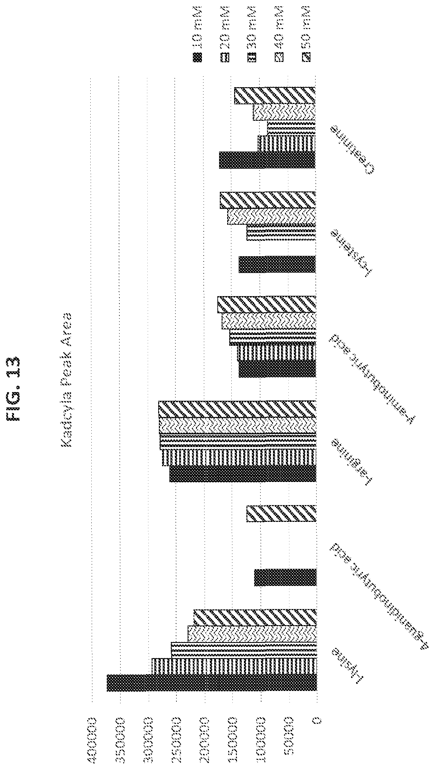

**FIG. 13** depicts peak areas for Kadcyla in exemplary chromatographic separations on a prototype polyethylene oxide (PEO) bonded SEC column packed with 1.7 µm particles with average pore sizes of 270 Å, with a mobile phase comprising aqueous sodium phosphate buffer and varying concentrations of L-lysine, 4-guanidinobutyic acid, L-arginine, gamma-aminobutyric acid, L-cysteine, or creatinine.

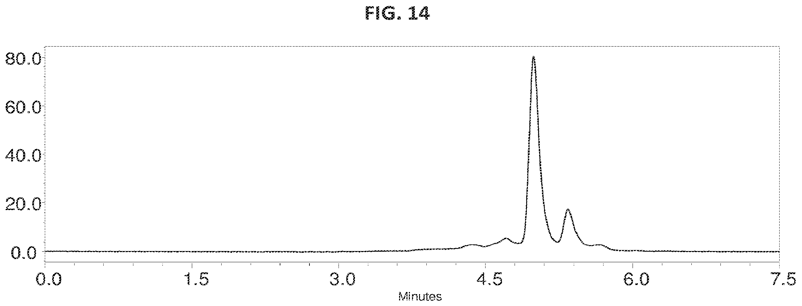

**FIG. 14** depicts an exemplary chromatographic separation of replication-incompetent human adenovirus type 5 on a prototype SEC column packed with 3 µm particles with an average pore size of 2000 Å, modified at least in part with a OH-terminated polyethylene glycol (PEG) bonding, with a mobile phase comprising aqueous sodium phosphate buffer, sodium chloride, and 30 mM L-arginine.

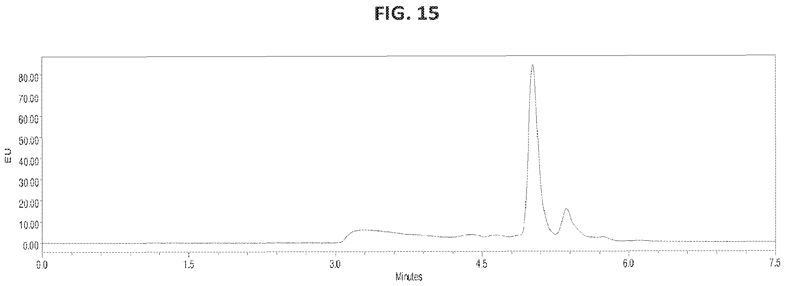

**FIG. 15** depicts an exemplary chromatographic separation of replication-incompetent human adenovirus type 5 on a prototype SEC column packed with 3 µm particles with an average pore size of 2000 Å, modified at least in part with a OH-terminated polyethylene glycol (PEG) bonding, with a mobile phase comprising sodium phosphate buffer, sodium chloride, and arginine.

### DETAILED DESCRIPTION

Before describing several example embodiments of the technology, it is to be understood that the technology is not limited to the details of construction or process steps set forth in the following description. The technology is capable of other embodiments and of being practiced or being carried out in various ways.

### Definitions

With respect to the terms used in this disclosure, the following definitions are provided. This application will use the following terms as defined below unless the context of the text in which the term appears requires a different meaning.

The articles "a" and "an" are used herein to refer to one or to more than one (i.e., to at least one) of the grammatical object of the article. The term "about" used throughout this specification is used to describe and account for small fluctuations. For example, the term "about" can refer to less than or equal to ±5%, such as less than or equal to ±2%, less than or equal to ±1%, less than or equal to ±0.5%, less than or equal to ±0.2%, less than or equal to ±0.1% or less than or equal to ±0.05%. All numeric values herein are modified by the term "about," whether or not explicitly indicated. A value modified by the term "about" of course includes the specific value. For instance, "about 5.0" must include 5.0.

Chromatography is a separation method for concentrating or isolating one or more compounds (e.g., biomolecules) found in a mixture. The compounds (e.g., biomolecules) are normally present in a sample. This disclosure uses the term "sample" broadly to represent any mixture which an individual may desire to analyze. The term "mixture" is used in the sense of a fluid containing one or more dissolved compounds (e.g., biomolecules). A compound of interest present in said sample is referred to as an analyte.

Chromatography is a differential migration process. Compounds in a mixture traverse a chromatographic column at different rates, leading to their separation. The migration occurs by convection of a fluid phase, referred to as the mobile phase, in relationship to a packed bed of particles or a porous monolith structure, referred to as the stationary phase. In some modes of chromatography, differential migration occurs by differences in affinity of analytes with the stationary phase and mobile phase.

Size exclusion chromatography (SEC) is a type of chromatography in which the analytes in a mixture are separated or isolated on the basis of hydrodynamic radius. In SEC, separation occurs because of the differences in the ability of analytes to probe the volume of the porous stationary phase media. See, for example, A. M. Striegel et. al. Modern Size-Exclusion Chromatography: Practice of Gel Permeation and Gel Filtration Chromatography, 2nd Edition, Wiley, NJ, 2009. SEC is typically used for the separation of large molecules or complexes of molecules. For example, without limitation, many large molecules of biological origin ("biomolecules"), such as deoxyribonucleic acids (DNAs), ribonucleic acids (RNAs), proteins, polysaccharides, antibody-drug conjugates, and fragments and complexes of any thereof are analyzed by SEC. Synthetic polymers, plastics, and the like are also analyzed by SEC.

SEC is normally performed using a column having a packed bed of particles. The packed bed of particles is a separation media or stationary phase through which the mobile phase will flow. The column is placed in fluid communication with a pump and a sample injector. The sample is loaded onto the column under pressure by the sample injector and the sample components and mobile phase are pushed through the column by the pump. The components in the sample leave or elute from the column with the largest molecules (largest hydrodynamic radius) exiting first and the smallest molecules leaving last.

The column is placed in fluid communication with a detector, which can detect the change in the nature of the mobile phase as the mobile phase exits the column. The detector will register and record these changes as a plot, referred to as a chromatogram, which is used to determine the presence or absence of the analyte, and, in some embodiments, the concentration thereof. The time at which the analyte leaves the column (retention time) is an indication of the size of the molecule. Molecular weight of the molecules can be estimated using standard calibration curves. Examples of detectors used for SEC are, without limitation, refractive index detectors, UV detectors, light-scattering detectors, and mass spectrometers.

"Hybrid", including "inorganic-organic hybrid material," includes inorganic-based structures wherein an organic functionality is integral to both the internal or "skeletal" inorganic structure as well as the hybrid material surface. The inorganic portion of the hybrid material may be, e.g., e.g., alumina, silica, titanium, cerium, or zirconium or oxides thereof, or ceramic material. "Hybrid" includes inorganic-based structures wherein an organic functionality is integral to both the internal or "skeletal" inorganic structure as well as the hybrid material surface. Exemplary hybrid materials are shown in U.S. Pat. Nos. 4,017,528, 6,528,167, 6,686,035, and 7,175,913. One non-limiting example of an inorganic-organic hybrid material is an ethylene-bridged hybrid material having an empirical formula of SiO₂(O_{1.5}SiCH₂CH₂SiO_{1.5})_{0.25}.

The terms "polyethylene glycol" and "polyethylene oxide" are used synonymously herein, both terms referring to oligomeric or polymeric polyether compounds having the formula -(O-CH₂CH₂)ₙ-OH. Accordingly, the abbreviations for "polyethylene glycol" and "polyethylene oxide", "PEG" and "PEO", respectively, are used synonymously herein.

The term "methoxy-terminated polyethylene glycol", abbreviated herein as "MeO-PEO" or MeO-PEG", refers to oligomeric or polymeric polyether compounds having the formula -(O-CH₂CH₂)ₙ-OMe. In contrast to hydroxy-terminated polyethylene glycols (HO-PEGs), MeO-PEGs do not have a free hydroxyl (OH) group available, having been capped with a methyl group.

The term "surface modification" as used herein, refers to the process of modifying the surface of a material by changing physical and/or chemical characteristics of the surface to improve the properties. The term "surface modified" as used herein, refers to a material (e.g., a porous stationary phase particle or core material) which has been reacted with a surface modifying group (a "surface modifier") to covalently bond, non-covalently bond, adsorb, or otherwise attach the surface modifier to the surface of the core material, or the surface of the stationary phase material. In certain embodiments, the surface modifying group is attached to the surface of the material by a siloxane bond. For example, the surface of a silica or hybrid silica material contains silanol groups, which can be reacted with a reactive organosilane (e.g., halo or alkoxy substituted silane), thus producing a Si-O-Si-C linkage. The surface modification can be a bonded surface or a coated surface.

The term "bonded surface" refers to a material (e.g., a porous stationary phase particle or core material) which has a monolayer of covalently attached silane molecules as a result of a bonding reaction between the surface modifying group and available hydroxyl groups on the surface of the material.

The term "coated surface" refers to a material (e.g., a porous stationary phase particle or core material) which has multilayers of the surface modifying group(s) due to oligomer and polymer formation of the surface modifying group(s) and horizontal and vertical polymerization reactions on the surface of the material.

The phrase "at least some substantial portion" as used herein to describe the extent of modification (i.e., bonding or coating), means that the surface density of the modification (e.g., a hydroxy-terminated polyethylene glycol) on the surface of the stationary phase particles is a minimum of about 0.5 micromole per square meter of particle surface area (0.5 µmol/m²). Surface density of the modification may be determined by calculating the difference in % carbon of the particle before and after the surface modification, as measured by elemental analysis. Surface density as reported herein is determined according to this calculation.

Reference herein to the "surface" of the stationary phase particles is, unless otherwise indicated or contradicted by the context, intended to mean the outermost extent of the particle surface.

Embodiments of the present disclosure are now described in detail as methods for performing SEC with the understanding that such methods are exemplary methods. Such methods constitute what the inventors now believe to be the best mode of practicing the technology. Those skilled in the art will recognize that such methods are capable of modification and alteration.

### Methods of Performing Size Exclusion Chromatography

Disclosed herein is a method for performing size exclusion chromatography (SEC). The method generally comprises contacting a sample containing at least one analyte with an immobilized stationary phase within a column, flowing a mobile phase through the immobilized stationary phase for a period of time, and eluting the at least one analyte from the immobilized stationary phase in said mobile phase.

Typically, methods of performing SEC for separation of proteinaceous analytes utilize a mobile phase comprising a buffer and a salt, and may include mild chaotropes, surfactants, or organic solvents. A mobile phase composition works to keep the analyte in its native form, prevent or reduce aggregation, and yield a quality separation and peak shape. Undesired (e.g., hydrophobic) interactions leading to poor chromatography are generally mitigated through mobile phase optimization, particularly utilizing various salts or organic co-solvents in a variety of concentrations in an attempt to reduce ionic and hydrophobic secondary interactions. However, such optimization is not always straightforward, and increasing the salt concentration or adding organic co-solvents can induce aggregation or denaturation, leading to a decrease in native monomer. Further, the addition of high concentrations of salts can exacerbate hydrophobic interactions. For example, a mobile phase with sufficient ionic strength to ensure analyte stability and solubility can inadvertently cause secondary interactions, leading to poor peak shape and recovery. The problem of hydrophobic interactions can be most easily evidenced when separating analytes with hydrophobic moieties such as antibody drug conjugates (ADCs).

The use of the amino acid arginine has previously been reported as an alternative mobile phase additive, or in combination with other mobile phase additives, for improving chromatography. See, e.g., Yumioka, et al., J Pharm Sci 2010, 99 (2), 618-20; Ejima et al., Journal of Chromatography A 2005, 1094 (1), 49-55; and US Patent No. 7,501,495 to Ajinomoto Co.). The presence of such amino acids is thought to help reduce aggregate formation through the inhibition of protein-protein interactions. See, e.g., Schneider et al., J Phys Chem B 2011, 115 (22), 7447-7458). However, effects on protein aggregation require high concentrations on the order of 200-500 mM. Such high amino acid concentrations would place a burden on many conventional chromatographic detectors, including mass spectrometers, and would likely require an additional sample clean up step, making use of amino acid additives less suitable as mobile phase modifiers.

Surprisingly, according to the present disclosure, it has been found that when using novel PEO bonded or coated SEC particles in the stationary phase which reduce secondary interactions, the threshold for improved chromatography (e.g., better peak shape and peak area) with amino acid supplementation of the mobile phase is well below the concentration required for protein aggregate stabilization. Accordingly, in one aspect is provided a method for performing size exclusion chromatography on a sample containing at least one analyte, the method comprising:
a. contacting said sample with a column chromatography device comprising a column having an interior for accepting a stationary phase, and an immobilized stationary phase within said interior of the column, wherein the immobilized stationary phase comprises porous particles having a diameter with a mean size distribution of between about 1 and about 20 µm; an average pore size from about 40 to about 3000 Å; and wherein said porous particles are surface modified with a hydroxy-terminated polyethylene glycol at a surface concentration from about 0.5 to about 5.0 µmoles/m²;
b. flowing a mobile phase through the immobilized stationary phase for a period of time, the mobile phase comprising water; a buffer; and an amino acid or derivative thereof, wherein the amino acid or derivative thereof is present in the mobile phase at a concentration from about 5 to about 40 mM; and
c. eluting the at least one analyte from the immobilized stationary phase in the mobile phase.

Each of the components of the disclosed method are described further herein below.

### Analyte

The method for performing size exclusion chromatography as disclosed herein comprises a sample containing at least one analyte. Notably, the utility of the presently disclosed method is not limited to biopharmaceuticals or proteinaceous analytes. In some embodiments, the at least one analyte comprises a small molecule drug, a natural product, or a polymer. In some embodiments, the at least one analyte comprises one or more biomolecules. In some embodiments, the biomolecule is a nucleic acid (e.g., RNA, DNA, oligonucleotide), protein (e.g., fusion protein), peptide, antibody (e.g., monoclonal antibody (mAb)), antibody-drug conjugate (ADC), polysaccharides, virus, virus-like particle, viral vector (e.g., gene therapy viral vector, adeno-associated viral vector), biosimilar, or any combination thereof. In some embodiments, the at least one analyte comprises a nucleic acid, a polysaccharide, a peptide, a polypeptide, a protein, or any combination thereof. In some embodiments, the at least one analyte comprises an adenovirus, an adeno-associated virus, mRNA, DNA, a plasmid, an exosome, an extracellular vesicle, a lipid nanoparticle encapsulated nucleic acid, or a combination thereof. In some embodiments, the at least one analyte comprises an adenovirus or an AAV. In some embodiments, the at least one analyte comprises an antibody. In some embodiments, the at least one analyte comprises a monoclonal antibody (mAb). In some embodiments, the at least one analyte comprises a high molecular weight species or aggregate form of an antibody. In some embodiments the at least one analyte is an antibody-drug conjugate.

### Mobile phase

The method for performing SEC as disclosed herein comprises flowing a mobile phase through an immobilized stationary phase for a period of time. The mobile phase comprises an amino acid or derivative thereof, water, and a buffer. In certain specific embodiments, the mobile phase and, optionally the sample, are provided by a high performance liquid chromatography (HPLC) system.

### Amino acids

Amino acids are molecules containing an amine group, a carboxylic acid group, and a side-chain that is specific to each amino acid. As used herein, the term "amino acid" includes the known, naturally occurring protein amino acids, which are referred to by both their common three letter abbreviation and full names. The term "amino acid" also includes stereoisomers and modifications of the naturally occurring protein amino acids, as well as including non-protein amino acids, post-translationally modified amino acids, enzymatically synthesized amino acids, derivatized amino acids, and the like.

In some embodiments, the amino acid is an alpha (α)-amino acid. α-amino acids have the generic formula H₂N-C_{α}H*R*-COOH, where *R* is a side chain moiety and the amino group is attached to the carbon atom immediately adjacent to the carboxylate group (i.e., the α-carbon). The various α-amino acids differ in the side-chain moiety that is attached to the α-carbon. The side group may include a charged group (positive, negative, or zwitterionic), a polar uncharged group, a non-polar (e.g., alkyl) group, a hydrophobic moiety, a cyclic group, an aromatic or, any combination thereof.

Other suitable types of amino acid include those where the amino group is attached to a different carbon atom. For example, beta (β)-amino acids, in which the carbon atom to which the amino group is attached is separated from the carboxylate group by one carbon atom, C_{β}. For example, while α-alanine has the formula H₂N-C_{α}H(CH₃)-COOH, β-alanine has the general formula H₂N-C_{β}H₂-C_{α}H₂-COOH (i.e., 3-aminopropanoic acid). Gamma (γ)-amino acids are amino acids in which the carbon atom to which the amino group attaches is separated from the carboxylate moiety by two carbon atoms. For example, γ-amino butyric acid has the formula, H₂N-C_{γ}H₂-C_{β}H₂-C_{α}H₂-COOH.

In some embodiments, the amino acid is a proteinogenic amino acid. The term "proteinogenic amino acid" means that the amino acid is one of the 20 amino acids which are encoded for and incorporated into proteins in nature. Such amino acids are generally referred to as "natural" amino acids, and have the L-stereochemistry. In some embodiments, the amino acid is alanine, arginine, asparagine, aspartic acid, cysteine, glutamic acid, glutamine, glycine, histidine, isoleucine, leucine, lysine, methionine, phenyl alanine, proline, serine, threonine, tryptophan, tyrosine, valine, or combinations thereof. In some embodiments, the amino acid is L-arginine. In some embodiments, the amino acid is L- lysine.

In some embodiments, the amino acid is a non-natural amino acid, or is a non-proteinogenic amino acid. Non-limiting examples of such amino acids which may be suitable include creatine, creatinine, 4-gaunidinobutyric acid, taurine, γ-amino butyric acid, and ornithine. In some embodiments, the amino acid is L-ornithine.

In some embodiments, the amino acid is selected from the group consisting of arginine, ornithine, lysine, and combinations thereof.

In some embodiments, the amino acid is an amino acid derivative. The term "derivative" as used herein includes any modification to or variation in portion of an amino acid, including modification of a naturally occurring amino acid side chain moiety. Modifications to an amino acid include, but are not limited to, esterification, alkylation, acylation, halogenation, sulfonation, nitration, and carboxylation.

In some embodiments, the amino acid derivative is an N-acylated amino acid.

In some embodiments, the amino acid derivative is an alkyl ester of the amino acid. In particular embodiments, the amino acid derivative is arginine methyl ester.

The concentration of the amino acid or derivative thereof in the mobile phase may vary. In some embodiments, the amino acid or derivative thereof is present in the mobile phase at a concentration from about 5 to about 50 mM, such as from about 5, about 10, about 20, or about 30, to about 40, or about 50 mM. In some embodiments, the amino acid or derivative thereof is present in the mobile phase at a concentration from about 5 to about 20 mM. In some embodiments, the amino acid or derivative thereof is present in the mobile phase at a concentration of about 10 mM. In embodiments where combinations of amino acids are utilized, such concentrations refer to the total, and not the individual concentrations.

### Buffer

The mobile phase comprises a buffer. Buffers serve to control the ionic strength and the pH of the mobile phase. Many different substances may be used as buffers depending on the nature of the analyte. Non-limiting examples of suitable buffers include phosphates, tris(hydroxymethyl)aminomethane, and acetates. In some embodiments, the buffer comprises phosphate. In some embodiments, the buffer comprises acetate. In some embodiments, the buffer is ammonium acetate. In some embodiments, the buffer is an alkali metal phosphate. In some embodiments, the buffer is a sodium or potassium phosphate. In some embodiments, the buffer is sodium phosphate monobasic, sodium phosphate dibasic, or a combination thereof.

The concentration of the buffer may vary depending on the desired pH and ionic strength of the mobile phase. In some embodiments, the buffer is present at a concentration from about 10 to about 100 mM, such as from about 10, about 20, about 20, about 40, or about 50, to about 60, about 70, about 80, about 90, or about 100 mM.

The pH of the mobile phase may vary. In some embodiments, the pH value of the mobile phase is from about 5.0 to about 8.0. In some embodiments, the pH value of the mobile phase is from about 6.0 to about 7.5. In some embodiments, the pH is from about 6.0, or about 6.5, to about 7.0, or about 7.5. In some embodiments, the pH is about 6.0, about 6.1, about 6.2, about 6.3, about 6.4, about 6.5, about 6.6, about 6.7, about 6.8. about 6.9, about 7.0, about 7.1, about 7.2, about 7.3, about 7.4, or about 7.5.

### Salts

In some embodiments, the mobile phase comprises a salt. As used herein, the term "salt" refers to an ionic compound comprising an alkali or alkaline earth metal and a halogen (e.g., fluoride, chloride, bromide, iodide). Undesired interactions can be mitigated through utilizing a salt to reduce ionic secondary interactions. However, increasing the salt concentration can induce aggregation and thus lead to a decrease in native monomer, and the addition of high concentrations of salt can exacerbate hydrophobic interactions, and complicates mobile phase optimization. When present, suitable salts include, but are not limited to, sodium chloride and potassium chloride. Suitable concentrations of salts in the mobile phase range from about 10 to about 200 mM.

In other embodiments, the mobile phase is free of salts. Surprisingly, according to the present disclosure, it was found that in some embodiments, the presence of salt was detrimental to the separation (e.g., detracted from peak shape and tailing). The absence of a salt is beneficial in reducing the complexity of mobile phase optimization.

### Co-solvents

In some embodiments, the mobile phase comprises an organic co-solvent. Organic co-solvents such as methanol, ethanol, isopropanol or acetonitrile are common additives to SEC mobile phases. When present, a co-solvent, such as acetonitrile, is generally present at less than about 15% by volume in the mobile phase. However, such co-solvents may result in protein denaturing of proteinaceous analytes. In some embodiments, the mobile phase is free of organic co-solvents. Surprisingly, according to the present disclosure, it was found that in some embodiments, the presence of an organic co-solvent was detrimental to the separation (e.g., detracted from peak shape and tailing). The elimination of the requirement for organic co-solvents is beneficial in reducing the complexity of mobile phase optimization. In some embodiments, the mobile phase does not include an organic co-solvent and does not include a salt. In other embodiments, the mobile phase comprises a co-solvent. In the case of PEO modified stationary phase surfaces as described herein, conformational changes of the polymer chains can occur depending on method conditions, which can result in an increase in hydrophobic character of these surfaces. In certain embodiments, for example, in the separation of antibody-drug conjugates, such increased hydrophobic character may result in poor peak shape and reduced resolution. Accordingly, in some embodiments, the mobile phase comprises an organic co-solvent in an amount up to about 15% by volume in the mobile phase. In some embodiments, the co-solvent is acetonitrile. In some embodiments, the co-solvent is isopropanol. In some embodiments, the isopropanol is present in an amount from about 5 to about 15% by volume.

### Conditions

### Flow rate

The separation method as disclosed herein may be conducted by flowing the mobile phase through the stationary phase at a variety of different flow rates, which may be determined by one of skill in the art based on scale, stationary phase particle size, difficulty of separation, and the like. In some embodiments, flowing the mobile phase through the immobilized stationary phase is performed at a flow rate from about 0.2 mL/min to about 3 mL/min. In certain embodiments, the flow rate is about 1 mL/min. In some embodiments, the flow rate is about 2 mL/min. In some embodiments, the flow rate is about 3 mL/min. In some embodiments, the flow rate is less than 1 mL/min, such as from about 0.05, about 0.1, about 0.2, about 0.3, about 0.4, or about 0.5, to about 0.6, about 0.7, about 0.8, about 0.9, or about 1 mL/min. In some embodiments, the flow rate is about 0.35 mL/min.

### Temperature

The temperature at which the chromatography is performed (i.e., column temperature) may vary. In some embodiments, the column temperature is from about 20 to about 50°C, such as about 20, about 25, about 30, about 35, about 40, about 45, or about 50°C. In some embodiments, the method as disclosed herein is insensitive to variations in column temperature, meaning retention time, peak shape and height, and analyte stability are maintained across a range of temperatures (e.g., from about 30 to about 50°C). In certain embodiments, higher monomer peak efficiency may be obtained using sub-ambient column temperatures. Accordingly, in some embodiments, the column temperature is less than about 45°C, less than about 35°C, or less than about 25°C, such as from about 15 to about 25°C, or about 20°C.

### Time

The time required for the SEC separation will vary depending on many factors, but will generally be less than about 60 minutes, less than about 50 minutes, less than about 40 minutes, less than about 30 minutes, less than about 20 minutes, less than about 10 minutes, less than about 5 minutes, less than about 4 minutes, less than about 3 minutes, less than about 2 minutes, or less than about 1 minute. In particular, the time will be determined by the elution time of the analyte of interest. In some embodiments, the retention time is reproducible from run to run, and is relatively unaffected by changes in temperature, pH, buffer concentration, and the like.

### Stationary Phase Material

The methods of the present disclosure utilize a stationary phase material. Such material can be composed of one or more particles, such as one or more spherical particles. The particles are generally spherical but can be any shape useful in chromatography.

The particles have a particle size or distribution of particle sizes. Particle size may be measured, e.g., using a Beckman Coulter Multisizer 3 instrument as follows. Particles are suspended homogeneously in a 5% lithium chloride methanol solution. A greater than 70,000 particle count may be run using a 30 µm aperture in the volume mode for each sample. Using the Coulter principle, volumes of particles are converted to diameter, where a particle diameter is the equivalent spherical diameter, which is the diameter of a sphere whose volume is identical to that of the particle. Particle size can also be determined by light microscopy.

The particles generally have a size distribution in which the average (mean) diameter is from about 1 to about 50 µm, such as from about 1, about 2, about 5, about 10, or about 20, to about 30, about 40, or about 50 µm. In some embodiments, the particles have a diameter with a mean size distribution from about 1 to about 20 µm. In some embodiments, the particles have a diameter with a mean size distribution from about 1.7 µm to about 5 µm. In some embodiments, the particles have a size distribution in which the average diameter is about 1.7 µm. In some embodiments, the particles have a size distribution in which the average diameter is about 3 µm.

The particles are generally porous, and may be fully porous or superficially porous. Porous materials have a pore size or a distribution of pore sizes. The average pore size (pore diameter) may vary depending on the intended analyte. As described in U.S. Pat. No. 5,861,110, pore diameter can be calculated from 4V/S BET, from pore volume, or from pore surface area.

The pore diameter is generally selected to allow free diffusion of molecules in the analyte and mobile phase so they can interact with the stationary phase.

In some embodiments, the porous particles have an average pore size from about 0 to about 3000 Å, or from about 40 to about 3000 Å. For example, the average pore size may be from about 40, about 50, about 60, about 70, about 80, about 90, or about 100, to about 200, about 300, about 500, about 1000, about 2000, or about 3000 Å. In some embodiments, the average pore size is from about 100 to about 300 Å. In some embodiments, the average pore size is about 125 Å. In some embodiments, the average pore size is about 200 Å. In some embodiments, the average pore size is about 250 Å. In some embodiments, the average pore size is about 270 Å. In some embodiments, the average pore size is about 900 Å. In some embodiments, the average pore size is from about 1000 to about 3000 Å, or from about 1000 to about 2000 Å. In some embodiments, the average pore size is about 1000 Å. In some embodiments, the average pore size is about 2000 Å.

The porous particles may comprise any suitable material. Suitable materials include, but are not limited to silica, inorganic/organic hybrid materials, and polymeric materials. In some embodiments, the porous particles comprise silica, an inorganic/organic hybrid material, or a polymer. In some embodiments, the porous particles comprise silica. In some embodiments, the porous particles comprise inorganic/organic hybrid materials. In some embodiments, the porous particles comprise or are inorganic-organic hybrid ethylene bridged particles having an empirical formula of SiO₂(O_{1.5}SiCH₂CH₂SiO_{1.5})_{0.25}. Such materials may be prepared in a sol-gel synthesis by the co-condensation of 1,2-bis(triethoxysilyl)ethane (BTEE) with tetraethyl orthosilicate (TEOS). Suitable procedures are reported in Wyndham et al., Analytical Chemistry 2003, 75, 6781-6788 and US Patent No. 6,686,035.

The porous particles have a surface, and at least some substantial portion of the surface is modified with a hydroxy-terminated polyethylene glycol. The coverage density of the hydroxy-terminated polyethylene glycol on the surface of the modified porous particles may vary. For example, in some embodiments, the hydroxy-terminated polyethylene glycol modifier is present on the surface of the porous particles at a density from about 0.5 to about 15 µmol/m². In some embodiments, the hydroxy-terminated polyethylene glycol modifier is present on the surface of the porous particles at a density from about 0.5 to about 5 µmol/m², or from about 1 to about 2.0 µmol/m².

In some embodiments, the porous particles have a hydroxy-terminated polyethylene glycol modified surface. In some embodiments, the hydroxy-terminated polyethylene glycol has the formula 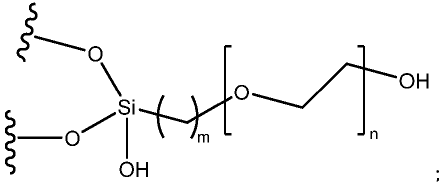 wherein:
m is an integer from about 1 to about 10;
n is an integer from about 2 to about 50; and
wherein the wavy lines indicate points of attachment to the surface of the porous particles.

Without wishing to be bound by theory, it is believed that the chain conformation of the polyethylene glycol unit on the surface depends at least partially on chain length.

In some embodiments, m is 1, 2, 3, 4, 5, 6, 7, 8, 9, or 10. In some embodiments, m is 2 or 3. In some embodiments, m is 3 (i.e., propyl).

In some embodiments, n is from about 2, about 5, about 10, about 15, or about 20, to about 25, about 30, about 35, about 40, about 45, or about 50. In some embodiments, n is from about 5 to about 15. In some embodiments, n is from about 8 to about 12. In particular embodiments, m is 3, and n is from about 8 to about 12. Such embodiments reflect the average chain length distribution in a commercially available polyethylene glycol useful in embodiments of the disclosure as a surface modifying reagent. In other embodiments, n may be a specific value, such as from about 8, about 9, or about 10, to about 11, or about 12.

In some embodiments, the hydroxy-terminated polyethylene glycol is bifunctional, forming a bridging ("bridged") polyethylene glycol when attached to the surface of the porous particle. In some embodiments, the bridging polyethylene glycol comprises a polyethylene glycol unit and further comprises two alkyl moieties, each having an exposed hydroxy group. In such embodiments, the exposed hydroxy group is the hydroxy termination of the hydroxy-terminated polyethylene glycol. In some embodiments, the bridged polyethylene glycol has the formula: 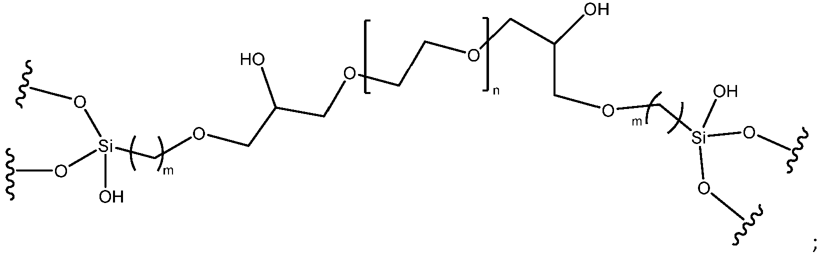 wherein the wavy lines indicate points of attachment to the surface of the porous particles, and m and n are each as defined above. In such embodiments, the modifier is a bis-(silylalkyl-2-hydroxy-alkoxy)polyethylene oxide. In some embodiments, m is 3, and n is 5 to 8.

In some embodiments, the hydroxy-terminated polyethylene glycol is attached directly to hydroxy groups on the initial surface (i.e., the native or as synthesized surface) of the porous particles. By initial surface, it is meant that the porous particle has not been treated with any coatings or bondings, and is in the native state as prepared. In such embodiments, the surface, following reaction with the hydroxy-terminated polyethylene glycol reagent, may be described as a bonded surface. A non-limiting depiction of a hydroxy-terminated polyethylene glycol bonded particle (**1**) is illustrated below:

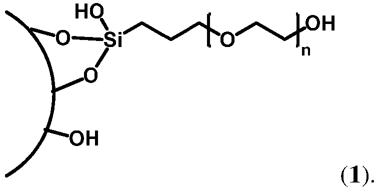

In other embodiments, the native or as synthesized surface is modified with a coating layer, either prior to or simultaneously with attachment of the hydroxy-terminated polyethylene glycol. In such embodiments, the hydroxy-terminated polyethylene glycol is attached through a complex network of silicon and oxygen bonds to the native surface of the porous particles.

In some embodiments, the hydroxy-terminated polyethylene glycol reagent is partially polymerized through hydrolytic condensation with itself, or with TEOS, prior to reaction with hydroxyl groups on the initial surface of the porous particle. In such embodiments, the resulting surface modified particle may be described as having a hydroxy-terminated polyethylene glycol coated surface. A non-limiting depiction of a hydroxy-terminated polyethylene glycol coated particle (**2**) is illustrated below:

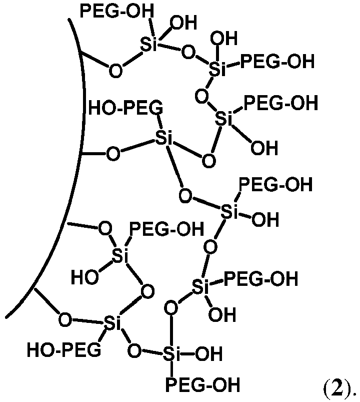

A non-limiting depiction of a hydroxy-terminated polyethylene glycol/TEOS coated particle (**3**) is illustrated below:

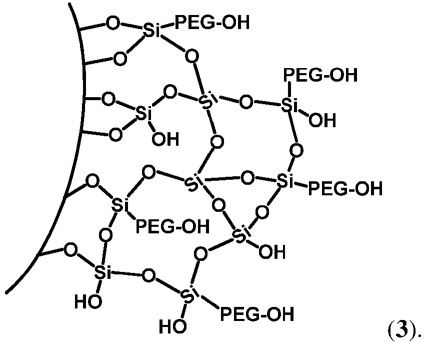

In some embodiments, the initial surface of the porous particle is coated with a silane reagent to form a secondary surface of oligomeric and/or polymeric siloxane multilayers on the particles. Such oligomeric and/or polymeric siloxane multilayers include those resulting from reaction of the particle surface with, for example, 1,2-bis(triethoxysilane)ethane (BTEE), tetraethyl orthosilicate (TEOS), or partial hydrolytic condensation products of BTEE and TEOS. The hydroxy-terminated polyethylene glycol reagent is then bonded on the coated surface. A non-limiting depiction of a hydroxy-terminated polyethylene glycol bonded and BTEE/TEOS coated particle (**4**) is illustrated below:

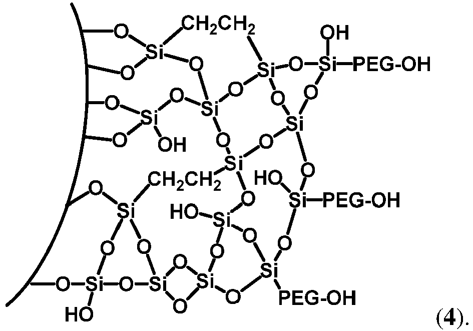

In some embodiments, the porous particles modified with a hydroxy-terminated polyethylene glycol further comprise a surface coating derived from reaction of the porous particle surface with BTEE, TEOS, or a partial hydrolytic condensation product of BTEE and TEOS.

In some embodiments, the porous particles comprise a surface coating derived from reaction of the porous particle surface with a partial hydrolytic condensation product of a hydroxy-terminated polyethylene glycol reagent, a partial hydrolytic condensation product of a hydroxy-terminated polyethylene glycol reagent with TEOS, or a combination thereof.

In some embodiments, the porous particles comprise or further comprise a surface coating derived from reaction of the porous particle surface with a partial hydrolytic condensation product of a polyethylene glycol silane reagent, a partial hydrolytic condensation product of a polyethylene glycol silane reagent with TEOS, or a combination thereof. Suitable polyethylene glycol-based reagents include, but are not limited to, bridging polyethylene glycol-based reagents as discussed above, and polyethylene glycol-based reagents having a masked or protected hydroxy group. In some embodiments, the hydroxy group, may be terminal or may be otherwise attached to the backbone of the reagent (e.g., an exposed hydroxy group on the carbon chain). In some embodiments wherein the hydroxy group is masked or protected, the masking or protecting group may be removed prior to performing chromatography with the particles bearing a surface modified with such reagents (i.e., providing the exposed or terminal hydroxy group). One of skill in the art will recognize such protecting groups and understand how to either maintain or remove them using standard chemical conditions known to one of skill in the art. A non-limiting set of suitable polyethylene glycol based silane reagents which may be used in addition to, or as an alternative to, the hydroxy-terminated polyethylene glycol reagent described herein above is provided in Table 1.

**Table 1. Example additional or alternative polyethylene glycol silane reagents**

| |
|---|
| 4,4,19,19-Tetraethoxy-3,7,10,13,16,20-hexaoxa-4,19-disiladocosane |
| BIS(3-TRIETHOXYSILYLPROPYL)POLYETHYLENE OXIDE (25-30 EO) |
| BIS-[3-(triethoxysilylpropoxy)-2-hydroxy-propoxy]polyethlyene oxide (5-8 EO) |
| N,N'-Bis-[(3-triethoxysilylpropyl)aminocarbonyl]polyethylene oxide (10-15 EO) |
| 4,4,19,19-Tetraethoxy-3,7,10,13,16,20-hexaoxa-4,19-disiladocosane |
| Poly(ethylene oxide), bis(trimethoxysilylpropyl) terminated |
| Poly(ethylene oxide), bis(triethoxysilylpropyl) terminated |
| 2-Propenoic acid, 2-methyl-, 2-[2-[3-(trichlorosilyl)propoxy]ethoxy]ethyl ester |
| 2-Propenoic acid, 2-methyl-, 9,9-dimethoxy-3,6,10-trioxa-9-silaundec-1-yl ester |
| 2-[3-(Dimethoxymethylsilyl)propoxy]ethyl 2-methyl-2-propenoate |
| 2-Propenoic acid, 2-methyl-, 10,10-dimethoxy-3,6,11-trioxa-10-siladodec-1-yl ester |
| 2-Propenoic acid, 2-methyl-, 2-[3-(chlorodimethylsilyl)propoxy]ethyl ester |
| 2-Propenoic acid, 2-methyl-, 2-[3-(chlorodipropylsilyl)propoxy]ethyl ester |
| 2-Propenoic acid, 2-methyl-, 2-[3-(diethoxyethylsilyl)propoxy]ethyl ester |
| 5,8,11-Trioxa-1-silatridecan-13-ol, 1,1,1-trichloro-, acetate |
| Ethanol, 2-[(chlorodimethylsilyl)methoxy]-, 1-acetate |
| 2,7,10,13-Tetraoxa-3-silapentadecan-15-ol, 3-methoxy-3-methyl-, acetate |
| 6,9,12,15,18,21-Hexaoxa-2-silatricosan-23-ol, 2-chloro-2-methyl-, acetate |
| Triethoxysilylpropoxy(Polyethyleneoxy) Dodecanoate |
| 2-[(Acetoxy(polyethyleneoxy)propyl]triethoxysilane |

In some embodiments, the porous particles having a hydroxy-terminated polyethylene glycol modified surface further comprise a surface bonding or coating derived from reaction of the hydroxy-terminated polyethylene glycol modified porous particles with a non-hydroxy-terminated polyethylene glycol reagent. In some embodiments, the reagent is selected from Table 1. In some embodiments, the reagent is 2-[methoxy(polyethyleneoxy)₆₋₉propyl]trichlorosilane or 2-[methoxy(polyethyleneoxy)₆₋₉propyl]tris(dimethylamino)silane.

In some embodiments, the porous particles having a hydroxy-terminated polyethylene glycol modified surface are hydroxy-terminated polyethylene glycol bonded. In some embodiments, the porous particles having a hydroxy-terminated polyethylene glycol modified surface are hydroxy-terminated polyethylene glycol coated. In some embodiments, the porous particles having a hydroxyl-terminated polyethylene glycol modified surface are hydroxy-terminated polyethylene glycol/TEOS coated. In some embodiments, the porous particles having a hydroxy-terminated polyethylene glycol modified surface are hydroxy-terminated polyethylene glycol bonded on a BTEE/TEOS coating.

**In** any of these embodiments, the porous particle so modified may further comprise a methoxy-terminated polyethylene glycol surface modification (e.g., bonding).. Non-limiting cartoon illustrations representative of possible configurations of such bonding and coating arrangements are provided below as structures **4, 5, 6,** and **7.** As one of skill in the art will recognize, such structures will possess a very complex network of silicon and oxygen bonds which cannot be adequately represented structurally. Accordingly, structures **4, 5, 6,** and **7** are provided merely to illustrate the general concept of the coating and bonding combinations disclosed herein. Structure **4** is representative of a hydroxy-terminated polyethylene glycol bonded BTEE/TEOS coated porous particle surface, as described herein above. Structure **5** is representative of a methoxy-terminated polyethylene glycol bonded and hydroxy-terminated polyethylene glycol coated porous particle surface. Structure **6** is representative of a methoxy-terminated polyethylene glycol bonded and hydroxy-terminated polyethylene glycol/TEOS coated porous particle surface. Structure **7** is representative of a hydroxy-terminated polyethylene glycol bonded and methoxy-terminated polyethylene glycol modified porous particle surface.

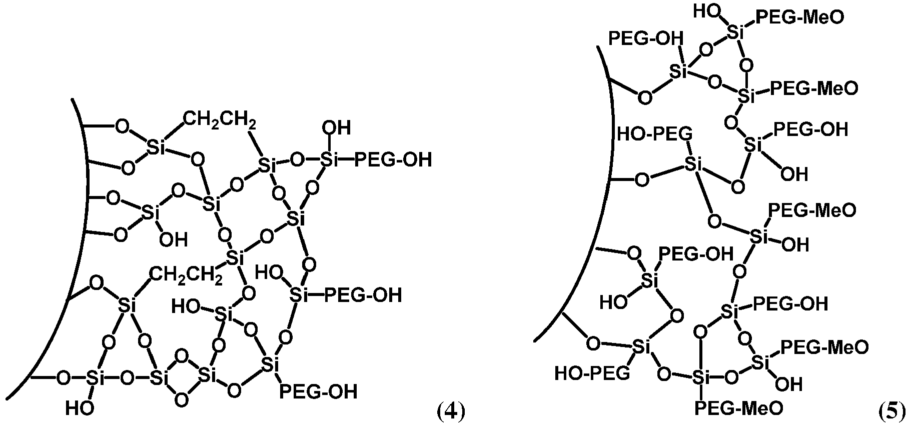

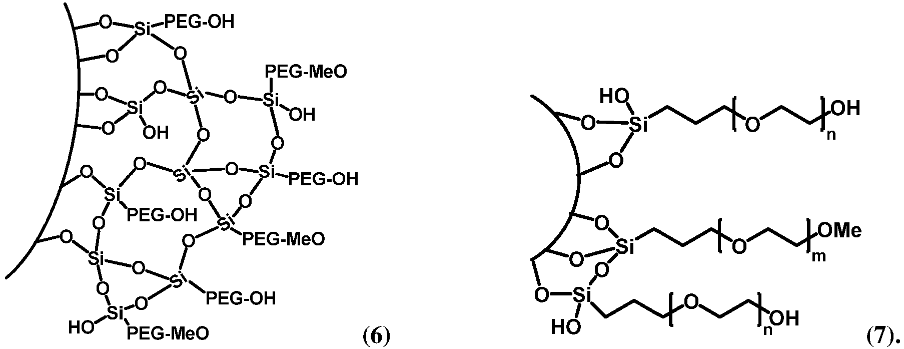

In some embodiments, the porous particles are porous silica particles. In particular embodiments, the porous silica particles are hydroxy-terminated polyethylene glycol bonded, hydroxy-terminated polyethylene glycol coated, or hydroxy-terminated polyethylene glycol bonded and BTEE/TEOS coated, and are further surface modified with a methoxy-terminated polyethylene glycol reagent. Accordingly, in some embodiments, the porous silica particles are hydroxy-terminated polyethylene glycol bonded, hydroxy-terminated polyethylene glycol coated, or hydroxy-terminated polyethylene glycol bonded and BTEE/TEOS coated, and are further methoxy-terminated PEG modified.

In some embodiments, the methoxy-terminated PEG modifying reagent is a methoxy-terminated polyethylene glycol silane reagent. In some embodiments, the methoxy-terminated polyethylene glycol silane reagent has a formula 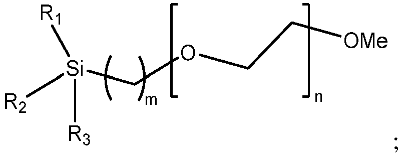 wherein:
at least one of R₁, R₂, and R₃ is OMe, OEt, Cl, or N(CH₃)₂;
m is an integer from about 1 to about 10; and
n is an integer from about 2 to about 20.

In some embodiments, m is 1, 2, 3, 4, 5, 6, 7, 8, 9, or 10. In some embodiments, m is 2 or 3. In some embodiments, m is 3 (i.e., propyl).

In some embodiments, n is from about 5 to about 15. In some embodiments, n is from about 6 to about 12, such as from about 6 to about 9.

In some embodiments, the methoxy-terminated PEG modifying reagent is 2-[methoxy(polyethyleneoxy)₆₋₉propyl]trichlorosilane or 2-[methoxy(polyethyleneoxy)₆₋₉propyl]tris(dimethylamino)silane.

Stationary phase materials surface modified with a hydroxy-terminated polyethylene glycol may be prepared, for example, by allowing the porous particles to react with a reagent such as trimethoxysilylpropyl polyethylene glycol, and hydrolyzing any remaining alkoxy groups. Some adjacent vicinal hydroxyl groups on the porous particle surface are at a distance such that difunctional reactions can occur between the vicinal hydroxyls and a difunctional or trifunctional reagent. When the adjacent hydroxyls on the surface are not suitably spaced for a difunctional reaction, then only a monofunctional reaction takes place.

The reaction is generally conducted according to standard methods, for example, by reaction of the porous particles with the appropriate reagent in an organic solvent under reflux conditions. An organic solvent such as toluene is typically used for this reaction.

In some embodiments, the stationary phase material is prepared by partially polymerizing the hydroxy-terminated polyethylene glycol reagent with itself through hydrolytic condensation prior to reaction with hydroxyl groups on the initial surface of the porous particle. Generally, incomplete (~50%) hydrolytic condensation reaction products may be obtained by reaction of the hydroxy-terminated polyethylene glycol reagent in ethanol (3.1 mol ethanol/mol silane) and 0.1 M HCl (13.5g/mol silane). In some embodiments, the hydroxy-terminated polyethylene glycol reagent is [hydroxy(polyethyleneoxy)₈₋₁₂propyl]triethoxysilane.

In some embodiments, the stationary phase material is prepared by partially polymerizing the hydroxy-terminated polyethylene glycol reagent with TEOS through hydrolytic condensation prior to reaction with hydroxyl groups on the initial surface of the porous particle. Generally, incomplete (~50%) hydrolytic condensation reaction products may be obtained by reaction of the hydroxy-terminated polyethylene glycol reagent with tetraethoxysilane (TEOS) (1:1 mol/mol) in ethanol (3.1 mol ethanol/mol silane) and 0.1 M HCl (13.5g/mol silane). In some embodiments, the hydroxy-terminated polyethylene glycol reagent is [hydroxy(polyethyleneoxy)₈₋₁₂propyl]triethoxysilane.

In each of the above embodiments describing a partial hydrolytic condensation product, the partial hydrolytic condensation product is then allowed to react with the porous particle, which may be a hybrid particle, a silica particle, or a hybrid or silica particle which has been coated with e.g., a BTEE/TEOS partial hydrolytic condensation product as described herein. For example, the BTEE/TEOS partial hydrolytic condensation product provides an inorganic/organic hybrid material coating on the silica particle. U.S. Utility Application Serial Number 16/082,823, published as US 2019/0091657A1 on March 28, 2019, describes coating a porous silica particle with an inorganic/organic hybrid material.

In some embodiments, the porous particles after modification to provide a hydroxy-terminated polyethylene glycol surface are reacted with an additional polyethylene glycol silane reagent. In some embodiments, the reagent is a methoxy-terminated polyethylene glycol silane reagent. In some embodiments, the reagent is 2-[methoxy(polyethyleneoxy)₆₋₉propyl]trichlorosilane or 2-[methoxy(polyethyleneoxy)₆₋₉propyl]tris(dimethylamino)silane. Generally, the method of reacting a hydroxy-terminated polyethylene glycol modified surface with the additional polyethylene glycol silane reagent comprises dispersing the porous particles (which may be a hybrid particle as described herein or silica particle) in a solvent and removing any residual water by azeotropic distillation.

In particular embodiments, the porous particles are silica particle having a pore size in a range of about 250 to about 3000 Å, or from about 1000 to about 2000 Å, and are hydroxy-terminated polyethylene glycol bonded, hydroxy-terminated polyethylene glycol coated, or hydroxy-terminated polyethylene glycol bonded and BTEE/TEOS coated, and are further surface modified with a methoxy-terminated polyethylene glycol reagent. Accordingly, in some embodiments, the porous silica particles are hydroxy-terminated polyethylene glycol bonded, hydroxy-terminated polyethylene glycol coated, or hydroxy-terminated polyethylene glycol bonded and BTEE/TEOS coated, and are further methoxy-terminated PEG modified.

In some embodiments, the methoxy-terminated PEG modifying reagent is a methoxy-terminated polyethylene glycol reagent. In some embodiments, the methoxy-terminated polyethylene glycol reagent has a formula 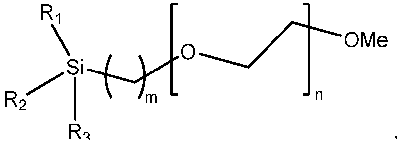 wherein:
at least one of R₁, R₂, and R₃ is OMe, OEt, Cl, or N(CH₃)₂;
m is an integer from about 1 to about 10; and
n is an integer from about 3 to about 20.

In some embodiments, m is 1, 2, 3, 4, 5, 6, 7, 8, 9, or 10. In some embodiments, m is 2 or 3. In some embodiments, m is 3 (i.e., propyl).

In some embodiments, n is from about 5 to about 15. In some embodiments, n is from about 6 to about 12, such as from about 6 to about 9.

In some embodiments, the methoxy-terminated PEG modifying reagent is 2-[methoxy(polyethyleneoxy)₆₋₉propyl]trichlorosilane or 2-[methoxy(polyethyleneoxy)₆₋₉propyl]tris(dimethylamino)silane.

The ratio of hydroxy-terminated polyethylene glycol groups to methoxy-terminated polyethylene glycol groups present on the porous particle surface may vary. For example, in some embodiments, the molar ratio is about 2:1, or about 1:1.

### Columns

For use in SEC, generally, the stationary phase will be immobilized in a housing having a wall defining a chamber, for example, a column having an interior for accepting the stationary phase. Such columns will have a length and a diameter.

In some embodiments, the length of the column is about 300 mm. In some embodiments, the length of the column is about 150 mm. In some embodiments, the length of the column is less than about 300 mm, less than about 150 mm, less than about 100 mm, or less than about 50 mm. In some embodiments, the length of the column is about 50 mm, about 30 mm, about 20 mm, or about 10 mm.

In some embodiments, the column has a bore size of about 4.6 mm inside diameter (i.d.). In some embodiments, the column has a bore size of greater than 4.6 mm i.d. In some embodiments, the column has a bore size of about 7.8 mm i.d. In some embodiments, the column has a bore size of greater than 7.8 mm i.d. In some embodiments, the column has a bore size of greater than about 4 mm i.d., greater than about 5 mm i.d., greater than about 6 mm i.d., or greater than about 7 mm i.d.

### Detecting

In some embodiments, the method further comprises detecting the presence or absence of the at least one analyte in the sample. Many suitable options exist for methods of detection. In some embodiments, the detecting is performed using a refractive index detector, a UV detector, a light-scattering detector, a mass spectrometer, or combinations thereof. In specific embodiments, the detecting is performed using a UV detector. Numerous detectors are available; however, a specific detector is a Waters ACQUITY^{®} UPLC^{®} Tunable UV Detector (Waters Corporation, Milford, Mass., USA).

### Reduction in Secondary Interactions

An ideal SEC separation would separate exclusively on the size, however, non-specific secondary interactions with the stationary phase reduces separation efficiency and reduces the quality of the separation. The most common secondary interactions are ionic and hydrophobic interactions, both of which result in poor chromatographic performance, including peak broadening, peak tailing, and loss of resolution and separation efficiency. At least two types of ionic interactions may occur. When a protein analyte and the stationary phase carry the same charge, ion-exclusion takes place due to electrostatic repulsion (decrease in protein elution time). When the protein and the stationary phase carry an opposite charge, ion-exchange takes place (increase in elution time). To ameliorate the ionic properties of the stationary phase surface, it is common practice to derivatize the material (e.g., silica) with hydrophobic silanes. The increase of hydrophobicity of the particle decreases the ionic interactions, but can introduce additional hydrophobic interactions. Antibody drug conjugates (ADCs) often have increased hydrophobicity compared to unmodified proteins due to their payload conjugations, ³ which can interact with the hydrophobic regions of a modified particle, resulting in poor quality separation. Other surface modifications (e.g., diol bonding, methoxy-terminated polyethylene glycol bonding) are know which ameliorate to varying degrees such interactions. Such undesired interactions can be mitigated through mobile phase optimization, particularly utilizing salt or organic co-solvent to reduce ionic and hydrophobic secondary interactions, respectively, though such optimization is not always straightforward. For example, a mobile phase with sufficient ionic strength to ensure analyte stability and solubility can inadvertently cause secondary interactions, leading to poor peak shape and recovery.

Surprisingly, it has been discovered according to the present disclosure that supplementing an SEC mobile phase with low concentrations of amino acids or derivatives thereof resulted in reduced secondary interactions between the analyte and the stationary phase material. Such reduction in secondary interactions is relative to SEC performed using a mobile phase which does not comprise an amino acid or derivative thereof. The reduction of the secondary interactions may be characterized by an improvement in one or more of peak shape, peak area, peak tailing, analyte recovery, or decreased inter-run variability. Such improvements can be quantified by calculation of factors such as USP tailing and asymmetry @4.4, and width at half height for analyte peaks.

In another aspect is provided a method for reducing a secondary interaction in size exclusion chromatography, the method comprising:
a. providing a sample including at least one analyte;
b. providing a column chromatography device configured to detect the presence or absence of least one analyte in a sample, the column chromatography device comprising a column having an interior for accepting a stationary phase, and an immobilized stationary phase within said column, wherein the immobilized stationary phase comprises porous particles having a diameter with a mean size distribution of between about 1 and about 20 µm; an average pore size from about 40 to about 2000 Å; and wherein said porous particles are surface modified with a hydroxy-terminated polyethylene glycol at a surface concentration from about 0.5 to about 5.0 µmoles/m²;
c. providing a mobile phase comprising water; a buffer; and an amino acid or derivative thereof, wherein the amino acid or derivative thereof is present in the mobile phase at a concentration from about 5 to about 40 mM;
d. injecting the sample onto the immobilized stationary phase;
e. flowing the mobile phase through the immobilized stationary phase for a period of time;
f. eluting the at least one analyte from the immobilized stationary phase in the mobile phase; and
g. detecting the presence of the least one analyte in the sample, wherein a peak in a chromatogram indicates the presence of the least one analyte in the sample, and wherein the reduction of the secondary interaction is characterized by an improvement in one or more of peak shape, peak area, peak tailing, analyte recovery, or decreased inter-run variability.

All methods described herein can be performed in any suitable order unless otherwise indicated herein or otherwise clearly contradicted by context. The use of any and all examples, or exemplary language (e.g., "such as") provided herein, is intended merely to better illuminate the materials and methods and does not pose a limitation on the scope unless otherwise claimed. No language in the specification should be construed as indicating any non-claimed element as essential to the practice of the disclosed materials and methods.

It will be readily apparent to one of ordinary skill in the relevant arts that suitable modifications and adaptations to the compositions, methods, and applications described herein can be made without departing from the scope of any embodiments or aspects thereof. The compositions and methods provided are exemplary and are not intended to limit the scope of the claimed embodiments. All of the various embodiments, aspects, and options disclosed herein can be combined in all variations. The scope of the compositions, formulations, methods, and processes described herein include all actual or potential combinations of embodiments, aspects, options, examples, and preferences herein.

Although the technology herein has been described with reference to particular embodiments, it is to be understood that these embodiments are merely illustrative of the principles and applications of the present technology. Thus, it is intended that the present technology include modifications and variations that are within the scope of the appended claims and their equivalents.

Reference throughout this specification to "one embodiment," "certain embodiments," "one or more embodiments" or "an embodiment" means that a particular feature, structure, material, or characteristic described in connection with the embodiment is included in at least one embodiment of the technology. Thus, the appearances of phrases such as "in one or more embodiments," "in certain embodiments," "in one embodiment" or "in an embodiment" in various places throughout this specification are not necessarily referring to the same embodiment of the technology. Furthermore, the particular features, structures, materials, or characteristics may be combined in any suitable manner in one or more embodiments. Any ranges cited herein are inclusive.

Aspects of the present technology are more fully illustrated with reference to the following examples. Before describing several exemplary embodiments of the technology, it is to be understood that the technology is not limited to the details of construction or process steps set forth in the following description. The technology is capable of other embodiments and of being practiced or being carried out in various ways. The following examples are set forth to illustrate certain aspects of the present technology and are not to be construed as limiting thereof.

### EXAMPLES

The present invention may be further illustrated by the following non-limiting examples describing the chromatographic devices and methods.

### Materials

All reagents were used as received unless otherwise noted. Those skilled in the art will recognize that equivalents of the following supplies and suppliers exist and, as such, the suppliers listed below are not to be construed as limiting.

The silica particles (1000 and 2000 Ȧ) were purchased from Daiso Fine Chem USA, INC (Daisogel; 3848 W Carson Street, Suite 105, Torrance, CA, 90503) and either used as received or treated with a dilute solution of acid (1M HCl, 20 h, 100 °C) before use.

Formulated ado-trastuzumab emtasine (Kadcyla, 2 mg/mL) was obtained from Genentech and diluted to 2-5 mg/mL concentration.

### Methods

The surface area (SA), pore volume (PV), and pore diameter (PD) of materials provided herein were measured using the multi-point N sorption method (Micromeritics ASAP 2400; Micromeritics Instruments Inc., Norcross, Ga.). The SA was calculated using the Brunauer-Emmett-Teller (BET) method, the PV was the single point value determined for P/Pd-0.98 to 0.99, and the PD was calculated from the desorption leg of the isotherm using the Barrett, Joyner, and Halenda (BJH) method. For average PD values above 500 Ȧ, the pore diameter and pore volume were measured by mercury porosimetry (Micromeritics AutoPore IV. Micromeritics, Norcross, Ga.). Skeletal densities were measured using a Micromeritics AccuPyc1330 Helium Pycnometer (V2.04N, Norcross, Ga.).

Particle sizes were measured using a Beckman Coulter Multisizer 3 analyzer (Miami, Fla.; 30-um aperture, 70,000 counts). The particle diameter (dp) was measured as the 50% cumulative diameter of the volume-based particle size distribution. The width of the distribution was measured as the 90% cumulative volume diameter divided by the 10% cumulative volume diameter (denoted 90/10 ratio).

The surface coverage was determined by the difference in particle % carbon before and after the surface modification, as measured by elemental analysis. Percent carbon (%C) and percent nitrogen (%N) values were measured by combustion analysis using a LECO TruMac carbon-nitrogen/sulfur Analyzer (Leco Corporation, Michigan, US).

A series of protoype (Examples 1-4 and 6-7) and reference (Example 5) stationary phase materials were prepared having different base particle materials and pore diameters. The base particles, modifications, and surface coverage are summarized below in Table 2.

### Example 1. Coated and Bonded Inorganic-Organic Hybrid Ethylene Bridged Particles, Average Pore Diameter of 270 Å.

A silane reagent (**1A**) was prepared from the incomplete (~50%) hydrolytic condensation of [hydroxy(polyethyleneoxy)₈₋₁₂propyl]triethoxysilane with tetraethyl orthosilicate (TEOS). To [hydroxy(polyethyleneoxy)₈₋₁₂propyl]triethoxysilane was added ethanol (3.1 mol ethanol/mol silane reagent), TEOS (1:1 molar ratio with the PEO reagent) and 0.1 M HCl (15.6 g/mol silane reagent). The solution was heated at 70°C for 18 h under an inert atmosphere. The reaction temperature was then increased to 90°C for atmospheric distillation to remove the ethanol. The temperature was then increased to 100°C for 1 h under an inert atmosphere. The reaction mixture was cooled to room temperature to obtain the product **1A.**

Inorganic-organic hybrid ethylene bridged particles (270 Å; prepared following the method as described in U.S. Patent 6,686,035) were fully dispersed in toluene (21 mL/g of particles). The surface area was 168 m²/g, and the pore volume was 1.19 cm³/g. The residual water was removed from the material by azeotropic distillation (110 °C, 1 h). The reaction temperature was held at 40°C while the silane reagent **1A** (1.0 g /g particle) was added and allowed to stir for 10 minutes. Catalytic aqueous NH₄OH was added (0.05-0.1 g /g particle). The reaction was stirred for an additional 10 minutes at 40°C, then increased to 60°C for 2 h. The reaction was then cooled to room temperature and the particles were isolated via filtration. The particles were subsequently washed twice with ethanol (10 ml/g) then dispersed in 70/30 (v/v) water/ethanol (10 mL/g). Ammonium hydroxide solution (1 g NH₄OH/g particle) was added, and the mixture was stirred at 50°C for 2 h. The reaction was then cooled < 40°C and the particles were isolated via filtration. The isolated particles were washed (10 ml/g) using the following sequence: 2 x methanol/water (1:1 v/v) and 2 x methanol. The isolated, surface modified particles were dried at 70°C for 16 h under vacuum. The process was repeated as needed to achieve the desired concentration of surface modifier.

To ensure uniformity of the PEO hybrid coating layer, the modified particles were exposed to elevated temperatures (100 - 140°C) and pH (8-9.8) following the hydrothermal treatment process according to the procedures reported in Jiang (U.S. Patent Nos. 6,686,035; 7,223,473; and 7,919,177) and Wyndham (International Patent Application Publication No. WO2008/103423).

The modified particles were then dispersed in 1.0 M HCl solution (8.4 mL/g particle) and the mixture was stirred at 100°C for 20 h. The reaction was then cooled below 40°C and the particles were isolated via filtration. The isolated particles were washed with water until the pH of the filtrate was higher than 5 and then washed with methanol x3. The isolated particles were dried at 70°C for 16 under vacuum. The isolated, surface modified particles were dried under vacuum at 70°C for 16 h. The surface coverage of the modified particles was 0.86 µmol/m².

The porous particles were fully dispersed in toluene (20 mL/g). The residual water was removed from the material by an azeotropic strip (110°C, 3 h). The reaction temperature was cooled below 40°C and 2-[methoxy(polyethyleneoxy)6-9propyl]tris(dimethylamino)silane (8 µmol/m²) was added. The reaction was stirred for 5 min and the temperature was increased to 110°C for 20 h. The reaction was then cooled to room temperature and the particles were isolated via filtration. The particles were subsequently washed using the following sequence: 7 x toluene, 1 x acetone, 6 x acetone/water (1:1 v/v), and 2 x acetone. The particles were then dispersed in a solution of acetone (8.2 mL/g particle) and 0.12 M ammonium acetate solution (1.8 mL/g particle) and the mixture was stirred at 59°C for 2 h. The reaction was then cooled below 40°C and the particles were isolated via filtration. The isolated particles were subsequently washed three times with acetone/water (1:1 v/v) and twice with acetone, then dried at 70°C for 16 h under vacuum. The surface coverage of the modified particles was 1.03 µmol/m². The hydroxy-terminated PEO bonded stationary phase particles were loaded in a 4.6x150 mm column.

### Example 2. Hydroxy-terminated PEO Bonded Inorganic-Organic Hybrid Ethylene Bridged Particles, Average Pore Diameter of 270 Å.

A stationary phase comprising hydroxy-terminated polyethylene oxide (PEO) bonded inorganic-organic hybrid ethylene bridged particles was prepared. The inorganic/organic hybrid particles with an empirical formula SiO₂(O_{1.5}SiCH₂CH₂SiO_{1.5})_{0.25} were synthesized in a sol-gel synthesis by the co-condensation of 1,2-bis(triethoxysilyl)ethane (BTEE) with tetraethyl orthosilicate (TEOS) using the procedures reported in Wyndham et al., Analytical Chemistry 2003, 75, 6781-6788 and US Patent No. 6,686,035. The obtained inorganic-organic hybrid ethylene bridged particles had an average particle size of 1.7 µm and an average pore diameter of 270 Å. The surface area was 171 m²/g, and the pore volume was 1.26 cm³/g.

The inorganic-organic hybrid ethylene bridged particles were then bonded to form the hydroxy-terminated PEO bonded stationary phase particles. The inorganic-organic hybrid ethylene bridged particles were dispersed in toluene (10 mL/g). The residual water was removed from the material by azeotropic distillation (110°C, 1-2 h). The reaction temperature was reduced below 40°C and concentrated hydrochloric acid (200 µL/g particles) was added, followed by [hydroxy(polyethyleneoxy)₈₋₁₂propyl]triethoxysilane (8 µmol/m²). The reaction was stirred for 5 min and the temperature was increased to 110°C for 20 h. The reaction was then cooled to RT and the particles were isolated via filtration. The particles were subsequently washed using the following sequence: 5 x toluene, 1 x acetone, 4 x acetone/water (1:1 v/v), and 2 x acetone.

Following the bonding reaction, hydrolysis of remaining ethoxysilyl groups was performed with ammonium acetate. The particles were dispersed in a mixture of acetone (8.2 mL/g particle) and 0.12 M ammonium acetate solution (1.8 mL/g particle), and the mixture was stirred at 59°C for 2 h. The reaction was then cooled < 40°C and the particles were isolated via filtration. The isolated particles were subsequently washed three times with acetone/water (1:1 v/v) and twice with acetone. The isolated, surface modified particles were dried under vacuum at 70°C for 16 h. The surface coverage of the hydroxy-terminated PEO was 1.73 µmol/m². The hydroxy-terminated PEO bonded stationary phase particles were loaded in a 4.6x150 mm column.

### Example 3. Hydroxy-terminated PEO Bonded Silica, Average Pore Diameter of 1000Å.

A stationary phase comprising hydroxy-terminated polyethylene oxide (PEO) bonded silica particles was prepared from silica particles having an average particle size of 3 µm and an average pore diameter of 1000 Å. The surface area was 28 m²/g, and the pore volume was 0.82 cm³/g.

The silica particles were dispersed in toluene (10 mL/g). The residual water was removed from the material by azeotropic distillation (110°C, 1-2 h). The reaction temperature was reduced below 40°C and concentrated hydrochloric acid (200 µL/g particles) was added, followed by [hydroxy(polyethyleneoxy)₈₋₁₂propyl]triethoxysilane (30 µmol/m²). The reaction was stirred for 5 min and the temperature was increased to 110°C for 20 h. The reaction was then cooled to RT and the particles were isolated via filtration. The particles were subsequently washed using the following sequence: 5 x toluene, 1 x acetone, 4 x acetone/water (1:1 v/v), and 2 x acetone.

Following the bonding reaction, hydrolysis of remaining ethoxysilyl groups was performed with ammonium acetate. The particles were dispersed in a mixture of acetone (8.2 mL/g particle) and 0.12 M ammonium acetate solution (1.8 mL/g particle), and the mixture was stirred at 59°C for 2 h. The reaction was then cooled < 40°C and the particles were isolated via filtration. The isolated particles were subsequently washed three times with acetone/water (1:1 v/v) and twice with acetone. The isolated, surface modified particles were dried under vacuum at 70°C for 16 h. The surface coverage of the hydroxy-terminated PEO was 1.46 µmol/m². The hydroxy-terminated PEO bonded stationary phase particles were loaded in a 4.6x150 mm column.

### Example 4. Coated and Bonded Silica, Average Pore Diameter of 1000 Å.

A silane reagent (**4A**) was prepared from the incomplete (~68%) hydrolytic condensation of 1,2-bis(triethoxysilane)ethane (BTEE) with tetraethyl orthosilicate (TEOS). To BTEE was added ethanol (3.1 mol ethanol/mol silane reagent), TEOS (1:4 molar ratio with the BTEE) and 0.1 M HCl (19.7 g/mol silane reagent). The solution was heated at 70°C for 18 h under an inert atmosphere. The reaction temperature was then increased to 90°C for atmospheric distillation to remove the ethanol. The temperature was then increased to 100°C for 1 h under an inert atmosphere. The reaction mixture was cooled to room temperature to obtain the condensation product **4A.**

Silica particles having an average particle size of 3 µm and an average pore diameter of 1000 Å were fully dispersed in toluene (21 mL/g of particles). The surface area was 28 m²/g, and the pore volume was 0.82 cm³/g. The residual water was removed from the material by azeotropic distillation (110 °C, 1 h). The reaction temperature was held at 40°C while the silane reagent **4A** (1.0 g/g particle) was added and allowed to stir for 10 minutes. Catalytic aqueous NH₄OH was added (0.05g /g particle). The reaction was stirred for an additional 10 minutes at 40°C, then increased to 60°C for 2 h. The reaction was then cooled to room temperature and the particles were isolated via filtration. The particles were subsequently washed twice with ethanol (10 ml/g) then dispersed in 70/30 (v/v) water/ethanol (10 mL/g). Ammonium hydroxide solution (1 g NH₄OH/g particle) was added, and the mixture was stirred at 50°C for 2 h. The reaction was then cooled < 40°C and the particles were isolated via filtration. The isolated particles were washed (10 ml/g) using the following sequence: 2 x methanol/water (1:1 v/v) and 2 x methanol. The isolated, surface modified particles were dried at 70°C for 16 h under vacuum. The process was repeated as needed to achieve the desired concentration of surface modifier.

To ensure uniformity of the coating layer, the modified particles were exposed to elevated temperatures (100-140°C) and pH (8-9.8) following the hydrothermal treatment process according to the procedures reported in Jiang (U.S. Patent Nos. 6,686,035; 7,223,473; and 7,919,177) and Wyndham (International Patent Application Publication No. WO2008/103423).

The modified particles were then dispersed in 1.0 M HCl solution (8.4 mL/g particle) and the mixture was stirred at 100°C for 20 h. The reaction was then cooled below 40°C and the particles were isolated via filtration. The isolated particles were washed with water until the pH of the filtrate was higher than 5 and then washed with methanol x3. The isolated, surface modified particles were dried under vacuum at 70°C for 16 h.

The porous coated silica particles were fully dispersed in toluene (20 mL/g). The residual water was removed from the material by an azeotropic strip (110 °C, 3 h). The reaction temperature was cooled below 40°C and concentrated hydrochloric acid (200 µL/g particle) was added, followed by [hydroxy(polyethyleneoxy)₈₋₁₂propyl]triethoxysilane (30 µmol/m²). The reaction was stirred for 5 min and the temperature was increased to 110°C for 20 h. The reaction was then cooled to room temperature and the particles were isolated via filtration. The particles were subsequently washed using the following sequence: 5 x toluene, 1 x acetone, 4 x acetone/water (1:1 v/v), and 2 x acetone. The particles were then dispersed in the solution of acetone (8.2 mL/g particle) and 0.12 M ammonium acetate solution (1.8 mL/g particle) and the mixture was stirred at 59°C for 2 h. The reaction was then cooled below 40°C and the particles were isolated via filtration. The isolated particles were subsequently washed three times with acetone/water (1:1 v/v) and twice with acetone, then dried at 70°C for 16 h under vacuum. The surface coverage of the modified particles was 1.03 µmol/m². The hydroxy-terminated PEO bonded stationary phase particles were loaded in a 4.6x150 mm column.

### Example 5. Reference BTEE/TEOS Coated Silica, Average Pore Diameter of 2000 Å.

A silane reagent (**4A**) was prepared from the incomplete (~68%) hydrolytic condensation of 1,2-bis(triethoxysilane)ethane (BTEE) with tetraethyl orthosilicate (TEOS). To BTEE was added ethanol (3.1 mol ethanol/mol silane reagent), TEOS (1:4 molar ratio with the BTEE) and 0.1 M HCl (19.7 g/mol silane reagent). The solution was heated at 70°C for 18 h under an inert atmosphere. The reaction temperature was then increased to 90°C for atmospheric distillation to remove the ethanol. The temperature was then increased to 100°C for 1 h under an inert atmosphere. The reaction mixture was cooled to room temperature to obtain the condensation product **4A.**

Silica particles having an average particle size of 3 µm and an average pore diameter of 2000 Å were fully dispersed in toluene (21 mL/g of particles). The surface area was 14 m²/g, and the pore volume was 0.69 cm³/g. The residual water was removed from the material by azeotropic distillation (110°C, 1 h). The reaction temperature was held at 40°C while the silane reagent **4A** (1.0 g /g particle) was added and allowed to stir for 10 minutes. Catalytic aqueous NH₄OH was added (0.05 g/g particle). The reaction was stirred for an additional 10 minutes at 40°C, then increased to 60°C for 2 h. The reaction was then cooled to room temperature and the particles were isolated via filtration. The particles were subsequently washed twice with ethanol (10 ml/g) then dispersed in 70/30 (v/v) water/ethanol (10 mL/g). Ammonium hydroxide solution (1 g NH₄OH/g particle) was added, and the mixture was stirred at 50°C for 2 h. The reaction was then cooled < 40°C and the particles were isolated via filtration. The isolated particles were washed (10 ml/g) using the following sequence: 2 x methanol/water (1:1 v/v) and 2 x methanol. The isolated, surface modified particles were dried at 70°C for 16 h under vacuum. The process was repeated as needed to achieve the desired concentration of surface modifier.

To ensure uniformity of the hybrid coating layer, the modified particles were exposed to elevated temperatures (100 - 140°C) and pH (8-9.8) following the hydrothermal treatment process according to the procedures reported in U.S. Patent Nos. 6,686,035, 7,223,473, and 7,919,177 to Jiang and International Patent Application Publication No. WO2008/103423 to Wyndham.

The modified particles were then dispersed in 1.0 M HCl solution (8.4 mL/g particle) and the mixture was stirred at 100°C for 20 h. The reaction was then cooled below 40°C and the particles were isolated via filtration. The isolated particles were washed with water until the pH of the filtrate was higher than 5 and then washed with methanol x3. The isolated, surface modified particles were dried under vacuum at 70°C for 16 h. The stationary phase particles were loaded in a 4.6x150 mm column.

### Example 6. Coated and Bonded Silica, Average Pore Diameter of 2000 Å.

Porous coated particles prepared according to Example 5 were fully dispersed in toluene (10 mL/g). The residual water was removed from the material by azeotropic distillation (110 °C, 1-2 h). The reaction temperature was reduced below 40°C and concentrated hydrochloric acid (200 µL/g particles) was added, followed by [hydroxy(polyethyleneoxy)₈₋₁₂propyl]triethoxysilane (40 µmol/m²). The reaction was stirred for 5 min and the temperature was increased to 110°C for 20 h. The reaction was then cooled to RT and the particles were isolated via filtration. The particles were subsequently washed using the following sequence: 5 x toluene, 1 x acetone, 4 x acetone/water (1:1 v/v), and 2 x acetone.

Following the bonding reaction, hydrolysis of remaining ethoxysilyl groups was performed with ammonium bicarbonate or ammonium acetate. The particles were dispersed in a mixture of acetone (8.2 mL/g particle) and 0.12 M ammonium acetate solution (1.8 mL/g particle), and the mixture was stirred at 59°C for 2 h. The reaction was then cooled < 40 °C and the particles were isolated via filtration. The isolated particles were subsequently washed three times with acetone/water (1:1 v/v) and twice with acetone. The isolated, surface modified particles were dried under vacuum at 70°C for 16 h. The surface coverage of the hydroxy-terminated PEG was 1.67 µmol/m². The coated, hydroxy-terminated PEG bonded stationary phase particles were loaded in a 4.6x150 mm column.

### Example 7. Hydroxy-terminated PEO Bonded Silica, Average Pore Diameter of 2000 Å.

A stationary phase comprising hydroxy-terminated polyethylene oxide (PEO) bonded silica particles was prepared from silica particles having an average particle size of 3 µm and an average pore diameter of 2000 Å. The surface area was 14 m²/g, and the pore volume was 0.69 cm³/g.

The silica particles were dispersed in toluene (10 mL/g). The residual water was removed from the material by azeotropic distillation (110 °C, 1-2 h). The reaction temperature was reduced below 40°C and concentrated hydrochloric acid (200 µL/g particles) was added, followed by [hydroxy(polyethyleneoxy)₈₋₁₂propyl]triethoxysilane (40 µmol/m²). The reaction was stirred for 5 min and the temperature was increased to 110°C for 20 h. The reaction was then cooled to RT and the particles were isolated via filtration. The particles were subsequently washed using the following sequence: 5 x toluene, 1 x acetone, 4 x acetone/water (1:1 v/v), and 2 x acetone.

Following the bonding reaction, hydrolysis of remaining ethoxysilyl groups was performed with ammonium bicarbonate or ammonium acetate. The particles were dispersed in a mixture of acetone (8.2 mL/g particle) and 0.12 M ammonium acetate solution (1.8 mL/g particle), and the mixture was stirred at 59°C for 2 h. The reaction was then cooled < 40 °C and the particles were isolated via filtration. The isolated particles were subsequently washed three times with acetone/water (1:1 v/v) and twice with acetone. The isolated, surface modified particles were dried under vacuum at 70°C for 16 h. The surface coverage of the hydroxy-terminated PEG was 1.56 µmol/m². The hydroxy-terminated PEG bonded stationary phase particles were loaded in a 4.6x150 mm column.

### Example 8. Hydroxy-terminated PEO Bonded/MeO-terminated PEG Modified Silica, Average Pore Diameter of 2000 Å.

Porous bonded particles prepared according to Example 7 were fully dispersed in toluene (20 mL/g). The residual water was removed from the material by an azeotropic strip (110°C, 3 h). The reaction temperature was cooled below 40°C and 2-[methoxy(polyethyleneoxy)₆₋₉propyl]tris(dimethylamino)silane (40 µmol/m²) was added. The reaction was stirred for 5 min and the temperature was increased to 110°C for 20 h. The reaction was then cooled to room temperature and the particles were isolated via filtration. The particles were subsequently washed using the following sequence: 7 x toluene, 1 x acetone, 6 x acetone/water (1:1 v/v), and 2 x acetone. The particles were then dispersed in the solution of acetone (8.2 mL/g particle) and 0.12 M ammonium acetate solution (1.8 mL/g particle) and the mixture was stirred at 59°C for 2 h. The reaction was then cooled below 40°C and the particles were isolated via filtration. The isolated particles were subsequently washed three times with acetone/water (1:1 v/v) and twice with acetone, then dried at 70°C for 16 h under vacuum. The surface coverage of the modified particles was 0.83 µmol/m². The hydroxy-terminated PEG bonded, methoxy-terminated PEG modified stationary phase particles were loaded in a 4.6x150 mm column.

**Table 2. Prototype Columns**

| Example # | Column Size | Particle Size/Pore Diameter | Base Particle Material | Surface Modification |
|---|---|---|---|---|
| 1 | 4.6 x 150 mm | 1.7µm, 270 Å, surface coverage 1.03 µmol/ m^{2;} 90/10=1.53 | Hybrid | HO-PEO(8-12 EO)propyltriethoxysilane/TEOS coated/MeO-PEO(6-9 EO)propyltris(dimethylamino) silane bonded |
| 2 | 4.6 x 150 mm | 1.7µm, 270 Å,90/10=1.53 | Hybrid | HO-PEO (8-12 EO)-propyltriethoxysilane bonded |
| 3 | 4.6 x 150 mm | 3 µm, 1000 Å | Silica | HO-PEO (8-12 EO)-propyltriethoxysilane bonded |
| 4 | 4.6 x 150 mm | 3 µm, 1000 Å | Silica | BTEE/TEOS coated/HO-PEO(8-12 EO)propyltriethoxysilane bonded |
| 5 (Reference) | 4.6 x 150 mm | 3 µm, 2000 Å | Silica | BTEE/TEOS coated |
| 6 | 4.6 x 150 mm | 3 µm, 2000 Å | Silica | BTEE/TEOS coated/HO-PEO(8-12 EO)propyltriethoxysilane bonded |
| 7 | 4.6 x 150 mm | 3 µm, 2000 Å | Silica | HO-PEO (8-12 EO)-propyltriethoxysilane bonded |
| 8 | 4.6 x 150 mm | 3 µm, 2000 Å | Silica | HO-PEG(8-12 EO)propyltriethoxysilane bonded/MeO-PEG(6-9 EO)propyltris(dimethylamino) silane modified |

### SEC Methods

### Example 9. Mobile Phase Supplemented with Lysine

Formulated Trastuzumab emtansine (Kadcyla, 2 mg/mL, Genentech) was diluted to 2 mg/mL and injected onto the column of Example 1 at a 1 µL injection volume. Separations were performed using a commercially available high performance liquid chromatography (HPLC) system (ACQUITY^{®} UPLC^{®} H-Class Bio system; available from Waters Corporation, Milford, MA) at a temperature of 30°C, and a flow rate of 0.35 mL/min. Detection was by UV absorption at 280 nm. The mobile phase components comprised A) 250 mM sodium phosphate buffer at pH of 6.8; B) 200 mM L-lysine; and D) water (18.2 megohm resistance). The mobile phase components were adjusted to provide a 40 mM concentration of sodium phosphate, a pH of 6.8, and 0, 10, 20, 30, 40, or 50 mM concentration of L-lysine.

When compared to a mobile phase consisting solely of 40 mM sodium phosphate pH 6.8, a significant improvement in terms of peak shape was observed (**FIG. 1**). With reference to **FIG. 1****,** the stacked chromatograms demonstrated that the presence of lysine improved analyte recovery by approximately two-fold when present between 10 and 50 mM. There was an increase in peak height, a decrease in tailing, and a significantly reduced peak width at half height. Additionally, there was a return to baseline, unlike with the mobile phase lacking L-lysine. Significantly, the peak area for the analyte, Kadcyla, was dramatically improved when the sodium phosphate buffer was supplemented with 10 mM L-lysine. The peak area actually reduced when the concentration of L-lysine was increased until it was indistinguishable from a separation performed solely with buffer as a mobile phase.

### Example 10. Mobile Phase Supplemented with Ornithine

Separations were performed as in Example 9, but substituting 200 mM L-ornithine for the L-lysine. Stacked chromatograms (**FIG. 2**) demonstrated that the presence of L-ornithine improved analyte recovery by approximately three-fold when present between 10 and 50 mM, with the 10 mM concentration exhibiting the best performance.

### Example 11. Mobile Phase Supplemented with Arginine

Separations were performed as in Example 9, but substituting 200 mM L-arginine for the L-lysine, and using the column of Example 2. Stacked chromatograms (**FIG. 3**) demonstrated that the presence of L-arginine improved analyte recovery when present between 10 and 50 mM. A plateau was reached at about 30 mM concentration.

### Example 12. Mobile Phase Supplemented with Arginine Methyl Ester

Separations were performed as in Example 9, but substituting 200 mM L-arginine methyl ester for the L-lysine. Stacked chromatograms (**FIG. 4**) demonstrated that the presence of L-arginine methyl ester improved analyte recovery by about three-fold when present between 10 and 50 mM. The results were comparable to those observed with supplementation of L-lysine or L-ornithine (Examples 1 and 2); the recovered peak area was significantly higher when the mobile phase was supplemented with 10 mM L-arginine methyl ester as opposed to the other tested concentrations.

### Example 13. Mobile Phase Supplemented with Arginine

Separations were performed as in Example 9, and adding an additional L-arginine concentration of 100 mM. The peaks were analyzed for tailing and peak width at 50% vs L-arginine concentration. Results in **FIGS. 5A** and **5B** demonstrated that the presence of L-arginine decreased tailing and peak width at half height at each concentration. The addition of L-arginine provided slightly different results compared to the previous Examples in that supplementing sodium phosphate with L-arginine with increasing concentrations did not lead to reduced peak areas. However, the improvement was observed to saturate at approximately 30 mM L-arginine. Without wishing to be bound by theory, is believed that this effect was due to the specific chemistry of the prototype SEC column.

### Example 14. Mobile Phase Supplemented with Arginine (BEH200 Column)

Separations were performed as in Example 13, but using a commercially available SEC column (BEH200; Waters Corporation, pore size of 200 Å, 1.7 µm, 4.6 mm x 150 mm). The peaks were analyzed for tailing and peak width at 50% vs L-arginine concentration. Results (**FIGS. 6A** and **6B**) demonstrated that the presence of L-arginine decreased tailing and peak width at half height for each concentration. The data demonstrated that a larger concentration of L-arginine was required to achieve optimal Kadcyla peak shape with the BEH column. Specifically, the effects appeared to be maximized at an L-arginine concentration of 50 to 100 mM.

### Example 15. Mobile Phase Supplemented with Arginine (BioSuite Column)

Separations were performed as in Example 14, but using a different commercially available SEC column (BioSuite; Waters Corporation, pore size of 250 Å, 10 µm silica particles, 1.7 µm, 7.5 mm x 300 mm). Stacked chromatograms (**FIG. 7**) demonstrated that the presence of L-arginine improved peak shape with increasing concentration. The data demonstrated that a significantly higher concentration (e.g., ≥100 mM) of L-arginine was required to generate a quality Kadcyla peak.

### Example 16. Mobile Phase Supplemented with Arginine; pH Study

Formulated Trastuzumab emtansine (Kadcyla, 2 mg/mL, Genentech) was diluted to 2 mg/mL and injected onto the column of Example 2 at a 1 µL injection volume. Separations were performed using a HClassBiol (Waters Corporation, Milford, MA) at a temperature of 30°C, and a flow rate of 0.35 mL/min. Detection was by UV absorption at 280 nm. The mobile phase components comprised A) 125 mM sodium phosphate monobasic buffer; B) 125 mM sodium phosphate dibasic buffer; C) 200 mM L-arginine; and D) water (18.2 megohm resistance). The mobile phase components were adjusted to provide a 40 mM concentration of sodium phosphate, a pH range from 6.0 to 7.5, and 30 mM concentration of L-arginine. Stacked chromatograms (**FIG. 8**) demonstrated that the favorable peak shape provided by supplementation of mobile phase with L-arginine occurred across the range of pH values.

### Example 17. Mobile Phase Supplemented with Arginine; pH Study (BEH200 Column)

Separations were performed as in Example 16, but using a commercially available SEC column (BEH200; Waters Corporation, pore size of 200 Å, 1.7 µm, 4.6 mm x 150 mm). Stacked chromatograms (**FIG. 9**) demonstrated that the presence of L-arginine improved peak shape across the range of pH values, with peak characteristics generally improving with increasing pH. Notably, the peak shape varied somewhat in comparison to the separation on the prototype polyethylene oxide (PEO) bonded SEC column (Example 8) over the same pH range. Without wishing to be bound by theory, it is believed that a greater concentration of L-arginine in the mobile phase may be required with such alternative conventional columns.

### Example 18. Mobile Phase Supplemented with Arginine; BEH200 Protein Standard

A BEH200 protein mixture standard (Waters, Inc.) containing thyroglobulin, IgG, BSA, Myoglobin, and uracil was injected onto the column of Example 2 at a 1 µL injection volume. Separations were performed using a HClassBio1 (Waters, Milford, MA) at a flow rate of 0.35 mL/min. Detection was by UV absorption at 280 nm. The mobile phase components comprised A) 250 mM sodium phosphate pH 6.8 buffer; B) 200 mM L-arginine; C) 1M sodium chloride; and D) water (18.2 megohm resistance). The mobile phase components were adjusted to provide three different mobile phases: 40 mM sodium phosphate at a pH of 6.8 (see **FIGS. 10A-E**); 40 mM sodium phosphate at a pH of 6.8 with 40 mM concentration of L-arginine (see **FIGS. 10F-10J**); and 40 mM sodium phosphate at a pH of 6.8 with 50 mM sodium chloride (see **FIGs. 10K-O**). The separations were performed at column temperatures of 30, 35, 40, 45, and 50°C. The results (**FIGS. 10A** to **10O**) demonstrated that supplementation of the mobile phase with L-arginine (see **FIGS. 10F-10J**) stabilized peak heights, shapes, and elution times across the range of temperatures. This was particularly noticeable for myoglobin.

### Example 19. Mobile Phase Supplemented with Arginine; Two Different Column Chemistries

Formulated Trastuzumab emtansine (Kadcyla, 2 mg/mL, Genentech) was diluted to 2 mg/mL and injected onto either the column of Example 3 or Example 4 at a 1 µL injection volume. Separations were performed using a HClassBiol (Waters, Milford, MA) at a temperature of 30°C, and a flow rate of 0.35 mL/min. Detection was by UV absorption at 280 nm. The mobile phase components comprised A) 250 mM sodium phosphate pH 6.8 buffer; B) 200 mM L-arginine; and D) water (18.2 megohm resistance). The mobile phase components were adjusted to provide a 40 mM concentration of sodium phosphate, and 0, 10, 20, 30, 40, or 50 mM concentration of L-arginine. Stacked chromatograms (**FIGS. 11A** and **11B**) demonstrated that the supplementation of mobile phase with L-arginine provided favorable peak characteristics, with a plateau at about 30 mM L-arginine concentration, for columns of Examples 3 and 4 (with and without hybrid coating, respectively). Both columns illustrated the observation in the prototype PEO bonded inorganic-organic hybrid ethylene bridged particles, i.e., that a concentration of greater than 30 mM L-arginine in the mobile phase provided no additional benefits regarding peak shape, peak area, or any other measurable chromatographic feature.

### Example 20. Mobile Phase Supplemented with Various Additives (1)

Formulated Trastuzumab emtansine (Kadcyla, 2 mg/mL, Genentech) was diluted to 2 mg/mL and injected onto the column of Example 1 at a 1 µL injection volume. Separations were performed using a HClassBio1 (Waters, Milford, MA) at a temperature of 30°C, and a flow rate of 0.35 mL/min. Detection was by UV absorption at 280 nm. The mobile phase components comprised A) 500 mM sodium phosphate pH 6.8 buffer; B) 200 mM poly-L-histidine or α-cyclodextrin at 5.01 g/100 ml); C) 200 mM gamma-aminobutyric acid, or poly-L-lysine; and D) water (18.2 megohm resistance). The mobile phase components were adjusted to provide a 40 mM concentration of sodium phosphate, and various concentrations of the additive. Chromatograms for separations performed with gamma-aminobutyric acid, poly-L-histidine, poly-L-lysine, or α-cyclodextrin (**FIGS. 12A, 12B****,** **12C**, and **12D**, respectively) demonstrated that the supplementation of mobile phase with these additives did not result in improved peak characteristics.

### Example 21. Mobile Phase Supplemented with Various Additives (2)

Separations were performed as in Example 20, but using as additives various concentrations (10-50 mM) of L-lysine, 4-gaunidinobutyric acid, L-arginine, gamma-aminobutyric acid, L-cysteine, or creatinine. The bar graph in **FIG. 13** demonstrated that the most favorable peak areas for the separation were obtained with lysine or arginine, and particularly with a low (e.g., 10 mM) concentration of lysine.

### Example 22. Adenovirus Separation on Hydroxy-Terminated PEO Bonded Stationary Phase with Arginine-Supplemented Mobile Phase

Size-based separations are of increasing importance to the emerging field of cell and gene therapy. High resolution, high throughput separations are needed to confirm the potency and safety of candidate therapeutics and vaccines. These advanced therapy medicinal products, as defined by the FDA and EMA, nearly exclusively correspond to large macromolecular complexes greater than 200 Å and sometimes upward of 2000 Å in diameter. It has been standard practice to measure the heterogeneity of these species using analytical ultracentrifugation (AUC). Because of the long turnaround times of producing AUC data, it could be said that the development of new modalities, including adeno-associated virus (AAV) and lentiviral vectored gene therapies, adenovirus vectored vaccines, and lipid nanoparticle mRNA, has been hindered. Accordingly, there is a need for size exclusion chromatography (SEC)-based assays that can more rapidly produce a size heterogeneity of these species without compromising the accuracy and fidelity of the measurement. To achieve this goal, high efficiency SEC columns with highly inert surfaces are required.

To determine the suitability of columns comprising the particles disclosed herein (3 µm, 2000 Å pore size, modified at least in part with a HO-terminated PEG bonding) to such separations, an adenovirus separation was performed. Specifically, a sample (5 µL) of replication-incompetent human adenovirus type 5 (packaged with a CMV-GFP plasmid; 1x10¹² pfu/mL) was size separated on a Waters H-Class Bio system using the column of Example 7 at a flow rate of 0.2 mL/min using a mobile phase comprising 40 mM sodium phosphate pH 7.0 buffer, 50 mM sodium chloride, and 30 mM arginine. The column temperature was 30°C. Detection was by native fluorescence using an ACQUITY FLR at an excitation wavelength of 260 nm and an emission wavelength of 350 nm at a scan rate of 10 Hz.

An exemplary chromatogram is provided in **FIG. 14****,** which shows that a monomer species was detected at a retention time of approximately 5 minutes. High molecular weight species were elucidated and observed eluting between 3 and 5 minutes, and there is fine structure seen in the profile that is indicative of dimer and trimer resolution as well as potential proteinaceous impurities. Two peaks eluted after the monomer species that are potentially attributable to incompletely formed or partially dissociated capsids, often observed in adenovirus preparations and formulations. Overall, the separation provided symmetrical, sharp peaks at a neutral pH and with a relatively low ionic strength.

### Example 23. Adenovirus Separation on Hydroxy-Terminated PEO Bonded Stationary Phase without Arginine in Mobile Phase

The use of an arginine-containing mobile phase is often beneficial to separations of macromolecules, but may not always be required to achieve an effective separation. To study the dependence on the presence of arginine for the separation performed in Example 22, the separation was performed in the absence of arginine in the mobile phase. An exemplary chromatogram is provided in **FIG. 15****,** which shows that non-ideal chromatographic behavior was encountered. Specifically, high molecular weight species show abnormal peak shapes and a tailing effect that would make this method unsuitable for drug product characterization.

## Claims

1. A method for performing size exclusion chromatography on a sample containing at least one analyte, the method comprising:
a. contacting said sample with a column chromatography device comprising a column having an interior for accepting a stationary phase, and an immobilized stationary phase within said interior of the column, wherein the immobilized stationary phase comprises porous particles having a surface and a diameter with a mean size distribution of between about 1 and about 20 µm; an average pore size from about 40 to about 3000 Å; and wherein said porous particles are surface modified with a hydroxy-terminated polyethylene glycol at a surface concentration from about 0.5 to about 5.0 µmoles/m²;
b. flowing a mobile phase through the immobilized stationary phase for a period of time, the mobile phase comprising water; a buffer; and an amino acid or derivative thereof, wherein the amino acid or derivative thereof is present in the mobile phase at a concentration from about 5 to about 40 mM;
c. eluting the at least one analyte from the immobilized stationary phase in the mobile phase; and
d. optionally, detecting the presence or absence of the at least one analyte in the sample.

2. The method of claim 1, wherein the amino acid or derivative thereof is present in the mobile phase at a concentration from about 5 to about 20 mM, preferably at a concentration of about 10 mM.

3. The method of any one of claims 1-2, wherein the amino acid or derivative thereof is:
an amino acid selected from the group consisting of L-arginine, L-ornithine, and L-lysine; or
an alkyl ester of an amino acid or an N-acylated amino acid, preferably wherein the alkyl ester of the amino acid is L-arginine methyl ester.

4. The method of any one of claims 1-3, wherein the at least one analyte comprises:
a nucleic acid, a polysaccharide, a peptide, a polypeptide, or a protein; and/or
an adenovirus, an adeno-associated virus (AAV), mRNA, DNA, plasmids, exosomes, extracellular vesicles, lipid nanoparticle encapsulated nucleic acids, or combinations thereof, preferably wherein the at least one analyte comprises an adenovirus or an AAV; and/or
an antibody; and/or
an antibody-drug conjugate.

5. The method of claim 1 comprising detecting the presence or absence of the at least one analyte in the sample, wherein the detecting is performed using a refractive index detector, a UV detector, a light-scattering detector, a mass spectrometer, or combinations thereof, preferably wherein the detecting is performed using a UV detector.

6. The method of any one of claims 1-5, wherein flowing the mobile phase through the immobilized stationary phase is performed at a flow rate from about 0.2 mL/min to about 3 mL/min.

7. The method of any one of claims 1-6, wherein the period of time is less than 60 minutes, less than 50 minutes, less than 40 minutes, less than 30 minutes, less than 20 minutes, less than 10 minutes, less than 5 minutes, less than 4 minutes, less than 3 minutes, less than 2 minutes, or less than 1 minute.

8. The method of any one of claims 1-7, wherein:
the buffer is present at a concentration of from about 10 to about 100 mM; and/or
the buffer is an alkali metal phosphate, preferably wherein the buffer is sodium phosphate monobasic, sodium phosphate dibasic, or a combination thereof, and/or
a pH value of the mobile phase is from about 6.0 to about 7.5; and/or
a column temperature is from about 20 to about 50°C.

9. The method of any one of claims 1-8, wherein the mobile phase does not include an organic co-solvent, does not include a salt, or does not include either of an organic co-solvent and a salt.

10. The method of any one of claims 1-8, wherein the porous particles comprise silica, an inorganic-organic hybrid material, or a polymer, preferably wherein the porous particles comprise inorganic-organic hybrid ethylene bridged particles having an empirical formula of SiO₂(O_{1.5}SiCH₂CH₂SiO_{1.5})_{0.25}.

11. The method of any one of claims 1-10, wherein the hydroxy-terminated polyethylene glycol has the formula: 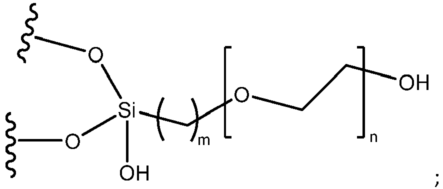 wherein:
m is an integer from about 1 to about 10;
n is an integer from about 2 to about 50; and
wherein the wavy lines indicate points of attachment to the surface of the porous particles.

12. The method of claim 11, wherein:
m is 2 or 3, preferably wherein m is 3 and n is from about 8 to about 12; and/or
n is from about 5 to about 15, or from about 8 to about 12.

13. The method of any one of claims 10-12, wherein the porous particles comprise silica, wherein the silica particles have an average pore size from 1000 to about 2000 Å, and wherein at least a portion of the surface of the porous silica particles is modified with a methoxy-terminated polyethylene glycol.

14. The method of claim 13, wherein the portion of the surface modified with the methoxy-terminated polyethylene glycol is the result of treatment of the porous silica particles with a methoxy-terminated polyethylene glycol reagent having a formula: 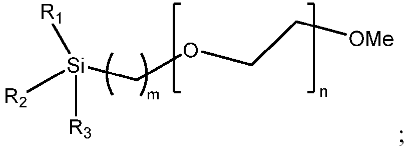 wherein:
at least one of R₁, R₂, and R₃ is OMe, OEt, Cl, or N(CH₃)₂;
m is an integer from about 1 to about 10; and
n is an integer from about 3 to about 20.

15. A method for reducing a secondary interaction in size exclusion chromatography, the method comprising:
a. providing a sample including at least one analyte;
b. providing a column chromatography device configured to detect the presence or absence of least one analyte in a sample, the column chromatography device comprising a column having an interior for accepting a stationary phase, and an immobilized stationary phase within said interior of the column, wherein the immobilized stationary phase comprises porous particles having a surface and a diameter with a mean size distribution of between about 1 and about 20 µm; an average pore size from about 40 to about 2000 Å; and wherein said porous particles are surface modifiedwith a hydroxy-terminated polyethylene glycol at a surface concentration from about 0.5 to about 5.0 µmoles/m²;
c. providing a mobile phase comprising water; a buffer; and an amino acid or derivative thereof, wherein the amino acid or derivative thereof is present in the mobile phase at a concentration from about 5 to about 40 mM;
d. injecting the sample onto the immobilized stationary phase;
e. flowing the mobile phase through the immobilized stationary phase for a period of time;
f. eluting the at least one analyte from the immobilized stationary phase in the mobile phase; and
g. detecting the presence of the least one analyte in the sample, wherein a peak in a chromatogram indicates the presence of the least one analyte in the sample, and wherein the reduction of the secondary interaction is **characterized by** an improvement in one or more of peak shape, peak area, peak tailing, analyte recovery, or decreased inter-run variability.

## Patentansprüche

1. Verfahren zum Durchführen einer Größenausschlusschromatographie an einer Probe, die mindestens einen Analyten enthält, wobei das Verfahren umfasst:
a. Inkontaktbringen der Probe mit einer Säulenchromatographievorrichtung, die eine Säule mit einem Innenraum zum Aufnehmen einer stationären Phase und einer immobilisierten stationären Phase in dem Innenraum der Säule umfasst, wobei die immobilisierte stationäre Phase poröse Teilchen umfasst, die eine Oberfläche und einen Durchmesser mit einer mittleren Größenverteilung zwischen ungefähr 1 und ungefähr 20 µm und eine durchschnittliche Porengröße von ungefähr 40 bis ungefähr 3000 Å aufweisen; und wobei die porösen Teilchen mit einem hydroxyterminierten Polyethylenglykol in einer Oberflächenkonzentration von ungefähr 0,5 bis ungefähr 5,0 µmol/m² oberflächenmodifiziert sind;
b. Fließenlassen einer mobilen Phase durch die immobilisierte stationäre Phase für eine Zeitdauer, wobei die mobile Phase Wasser, einen Puffer, und eine Aminosäure oder ein Derivat davon umfasst, wobei die Aminosäure oder das Derivat davon in der mobilen Phase in einer Konzentration von ungefähr 5 bis ungefähr 40 mM vorhanden ist;
c. Eluieren des mindestens einen Analyten aus der immobilisierten stationären Phase in die mobile Phase; und
d. gegebenenfalls Nachweisen der Anwesenheit oder Abwesenheit des mindestens einen Analyten in der Probe.

2. Verfahren nach Anspruch 1, wobei die Aminosäure oder ein Derivat davon in der mobilen Phase in einer Konzentration von ungefähr 5 bis ungefähr 20 mM, bevorzugt in einer Konzentration von ungefähr 10 mM, vorhanden ist.

3. Verfahren nach einem der Ansprüche 1 bis 2, wobei die Aminosäure oder deren Derivat ist: eine Aminosäure, die aus der Gruppe ausgewählt ist, die aus L-Arginin, L-Ornithin und L-Lysin ausgewählt ist; oder ein Alkylester einer Aminosäure oder einer N-acylierten Aminosäure, wobei der Alkylester der Aminosäure bevorzugt L-Argininmethylester ist.

4. Verfahren nach einem der Ansprüche 1 bis 3, wobei der mindestens eine Analyt umfasst:
eine Nukleinsäure, ein Polysaccharid, ein Peptid, ein Polypeptid oder ein Protein; und/oder
ein Adenovirus, ein Adena-assoziiertes Virus (AAV), mRNA, DNA, Plasmide, Exosomen, extrazelluläre Vesikel, in Lipid-Nanopartikel eingekapselte Nukleinsäuren oder Kombinationen davon, wobei der mindestens eine Analyt bevorzugt ein Adenovirus oder ein AAV umfasst; und/oder
einen Antikörper; und/oder
ein Antikörper-Wirkstoff-Konjugat.

5. Verfahren nach Anspruch 1, umfassend das Nachweisen des Vorhandenseins oder der Abwesenheit des mindestens einen Analyten in der Probe, wobei das Nachweisen unter Verwendung eines Brechungsindexdetektors, eines UV-Detektors, eines Lichtstreudetektors, eines Massenspektrometers oder Kombinationen davon durchgeführt wird, wobei das Nachweisen bevorzugt unter Verwendung eines UV-Detektors durchgeführt wird.

6. Verfahren nach einem der Ansprüche 1 bis 5, wobei das Fließenlassen der mobilen Phase durch die immobilisierte stationäre Phase mit einer Fließgeschwindigkeit von ungefähr 0,2 mL/min bis ungefähr 3 mL/min durchgeführt wird.

7. Verfahren nach einem der Ansprüche 1 bis 6, wobei die Zeitdauer weniger als 60 Minuten, weniger als 50 Minuten, weniger als 40 Minuten, weniger als 30 Minuten, weniger als 20 Minuten, weniger als 10 Minuten, weniger als 5 Minuten, weniger als 4 Minuten, weniger als 3 Minuten, weniger als 2 Minuten oder weniger als 1 Minute beträgt.

8. Verfahren nach einem der Ansprüche 1 bis 7, wobei:
der Puffer in einer Konzentration von ungefähr 10 bis ungefähr 100 mM vorliegt; und/oder
der Puffer ein Alkalimetallphosphat ist, wobei der Puffer bevorzugt ein einbasisches Natriumphosphat, ein zweibasisches Natriumphosphat oder eine Kombination davon ist; und/oder
ein pH-Wert der mobilen Phase ungefähr 6,0 bis ungefähr 7,5 beträgt; und/oder
eine Säulentemperatur von ungefähr 20 bis ungefähr 50°C beträgt.

9. Verfahren nach einem der Ansprüche 1 bis 8, wobei die mobile Phase kein organisches Co-Lösungsmittel enthält, kein Salz enthält oder weder ein organisches Co-Lösungsmittel noch ein Salz enthält.

10. Verfahren nach einem der Ansprüche 1 bis 8, wobei die porösen Teilchen Siliciumdioxid, ein anorganisch-organisches Hybridmaterial oder ein Polymer umfassen, wobei die porösen Teilchen bevorzugt anorganisch-organische ethylenverbrückte Hybridteilchen mit der empirischen Formel SiO₂(O_{1,5}SiCH₂CH₂SiO_{1,5})_{0,25} umfassen.

11. Verfahren nach einem der Ansprüche 1 bis 10, wobei das hydroxyterminierte Polyethylenglykol die Formel hat: 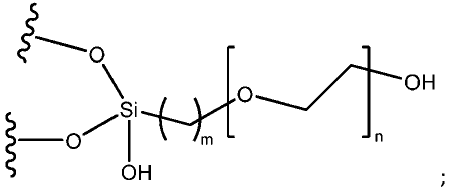 wobei:
m eine ganze Zahl von ungefähr 1 bis ungefähr 10 ist;
n eine ganze Zahl von ungefähr 2 bis ungefähr 50 ist; und
wobei die Wellenlinien Anhaftungspunkte an der Oberfläche der porösen Teilchen anzeigen.

12. Verfahren nach Anspruch 11, wobei:
m 2 oder 3 ist, wobei bevorzugt m 3 ist und n von ungefähr 8 bis ungefähr 12 ist; und/oder
n von ungefähr 5 bis ungefähr 15 oder von ungefähr 8 bis ungefähr 12 ist.

13. Verfahren nach einem der Ansprüche 10 bis 12, wobei die porösen Teilchen Siliciumdioxid umfassen, wobei die Siliciumdioxidteilchen eine durchschnittliche Porengröße von 1000 bis ungefähr 2000 Å aufweisen, und wobei mindestens ein Teil der Oberfläche der porösen Siliciumdioxidteilchen mit einem methoxyterminierten Polyethylenglykol modifiziert ist.

14. Verfahren nach Anspruch 13, wobei der mit dem methoxyterminierten Polyethylenglykol modifizierte Teil der Oberfläche das Ergebnis der Behandlung der porösen Siliciumdioxidteilchen mit einem methoxyterminierten Polyethylenglykol-Reagenz ist, das die Formel hat: 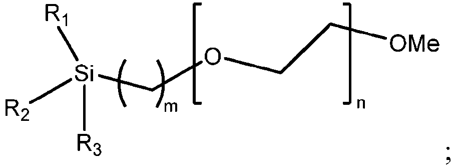 wobei:
mindestens eines von R₁, R₂ und R₃ OMe, OEt, CI oder N(CH₃)₂ ist;
m eine ganze Zahl von ungefähr 1 bis ungefähr 10 ist; und
n eine ganze Zahl von ungefähr 3 bis ungefähr 20 ist.

15. Verfahren zum Verringern einer sekundären Wechselwirkung in der Größenausschlusschromatographie, wobei das Verfahren umfasst:
a. Bereitstellen einer Probe, die mindestens einen Analyten enthält;
b. Bereitstellen einer Säulenchromatographievorrichtung, die dazu konfiguriert ist, das Vorhandensein oder die Abwesenheit mindestens eines Analyten in einer Probe nachzuweisen, wobei die Säulenchromatographievorrichtung eine Säule mit einem Innenraum zum Aufnehmen einer stationären Phase und einer immobilisierten stationären Phase in dem Innenraum der Säule umfasst, wobei die immobilisierte stationäre Phase poröse Teilchen umfasst, die eine Oberfläche und einen Durchmesser mit einer mittleren Größenverteilung zwischen ungefähr 1 und ungefähr 20 µm und eine durchschnittliche Porengröße von ungefähr 40 bis ungefähr 2000 Å aufweisen; und wobei die porösen Teilchen mit einem hydroxyterminierten Polyethylenglykol in einer Oberflächenkonzentration von ungefähr 0,5 bis ungefähr 5,0 µmol/m² oberflächenmodifiziert sind;
c. Bereitstellen einer mobilen Phase, die Wasser, einen Puffer und eine Aminosäure oder ein Derivat davon umfasst, wobei die Aminosäure oder das Derivat davon in der mobilen Phase in einer Konzentration von ungefähr 5 bis ungefähr 40 mM vorhanden ist;
d. Injizieren der Probe auf die immobilisierte stationäre Phase;
e. Fließenlassen der mobilen Phase durch die immobilisierte stationäre Phase für eine Zeitdauer;
f. Eluieren des mindestens einen Analyten aus der immobilisierten stationären Phase in die mobile Phase; und
g. Nachweisen des Vorhandenseins des mindestens einen Analyten in der Probe, wobei ein Peak in einem Chromatogramm das Vorhandensein des mindestens einen Analyten in der Probe anzeigt und wobei die Verringerung der sekundären Wechselwirkung durch eine Verbesserung von einem oder mehreren von der Peakform, der Peakfläche, des Peak-Tailings, der Analyt-Wiederfindung oder der verringerten Inter-Run-Variabilität gekennzeichnet ist.

## Revendications

1. Procédé pour effectuer une chromatographie d'exclusion stérique sur un échantillon contenant au moins un analyte, le procédé comprenant :
a. la mise en contact dudit échantillon avec un dispositif de chromatographie sur colonne comprenant une colonne ayant un intérieur pour recevoir une phase stationnaire, et une phase stationnaire immobilisée au sein dudit intérieur de la colonne, où la phase stationnaire immobilisée comprend des particules poreuses ayant une surface et un diamètre avec une distribution de taille moyenne d'entre environ 1 et environ 20 µm ; une taille de pore moyenne d'environ 40 à environ 3 000 Å ; et où lesdites particules poreuses sont modifiées en surface avec un polyéthylène glycol à terminaison hydroxy à une concentration en surface d'environ 0,5 à environ 5,0 µmoles/m² ;
b. la mise en écoulement d'une phase mobile à travers la phase stationnaire immobilisée pendant une période de temps, la phase mobile comprenant de l'eau ; un tampon ; et un acide aminé ou un dérivé de celui-ci, où l'acide aminé ou le dérivé de celui-ci est présent dans la phase mobile à une concentration d'environ 5 à environ 40 mM ;
c. l'élution de l'au moins un analyte à partir de la phase stationnaire immobilisée dans la phase mobile ; et
d. éventuellement, la détection de la présence ou de l'absence de l'au moins un analyte dans l'échantillon.

2. Procédé selon la revendication 1, où l'acide aminé ou le dérivé de celui-ci est présent dans la phase mobile à une concentration d'environ 5 à environ 20 mM, de préférence à une concentration d'environ 10 mM.

3. Procédé selon l'une quelconque des revendications 1 à 2, où l'acide aminé ou le dérivé de celui-ci est : un acide aminé choisi dans le groupe constitué par la L-arginine, la L-ornithine et la L-lysine ; ou un ester alkylique d'un acide aminé ou d'un acide aminé N-acylé, de préférence où l'ester alkylique de l'acide aminé est l'ester méthylique de la L-arginine.

4. Procédé selon l'une quelconque des revendications 1 à 3, où l'au moins un analyte comprend :
un acide nucléique, un polysaccharide, un peptide, un polypeptide ou une protéine ; et/ou
un adénovirus, un virus adéno-associé (AAV), un ARNm, un ADN, des plasmides, des exosomes, des vésicules extracellulaires, des acides nucléiques encapsulés dans des nanoparticules lipidiques, ou des combinaisons de ceux-ci, de préférence où l'au moins un analyte comprend un adénovirus ou un AAV ; et/ou
un anticorps ; et/ou un conjugué anticorps-médicament.

5. Procédé selon la revendication 1, comprenant la détection de la présence ou de l'absence de l'au moins un analyte dans l'échantillon, où la détection est effectuée à l'aide d'un détecteur d'indice de réfraction, d'un détecteur UV, d'un détecteur à diffusion de lumière, d'un spectromètre de masse ou de combinaisons de ceux-ci, de préférence où la détection est effectuée à l'aide d'un détecteur UV.

6. Procédé selon l'une quelconque des revendications 1 à 5, où la mise en écoulement de la phase mobile à travers la phase stationnaire immobilisée est effectuée à un débit d'environ 0,2 ml/min à environ 3 ml/min.

7. Procédé selon l'une quelconque des revendications 1 à 6, où la période de temps est inférieure à 60 minutes, inférieure à 50 minutes, inférieure à 40 minutes, inférieure à 30 minutes, inférieure à 20 minutes, inférieure à 10 minutes, inférieure à 5 minutes, inférieure à 4 minutes, inférieure à 3 minutes, inférieure à 2 minutes ou inférieure à 1 minute.

8. Procédé selon l'une quelconque des revendications 1 à 7, où :
le tampon est présent à une concentration d'environ 10 à environ 100 mM ; et/ou
le tampon est un phosphate de métal alcalin, de préférence où le tampon est du phosphate de sodium monobasique, du phosphate de sodium dibasique ou une combinaison de ceux-ci ; et/ou
une valeur de pH de la phase mobile est d'environ 6,0 à environ 7,5 ; et/ou
la température de la colonne est d'environ 20 à environ 50 °C.

9. Procédé selon l'une quelconque des revendications 1 à 8, où la phase mobile n'inclut pas de cosolvant organique, n'inclut pas de sel, ou n'inclut ni cosolvant organique ni sel.

10. Procédé selon l'une quelconque des revendications 1 à 8, où les particules poreuses comprennent de la silice, un matériau hybride inorganique-organique ou un polymère, de préférence où les particules poreuses comprennent des particules hybrides inorganiques-organiques pontées par de l'éthylène ayant une formule empirique de SiO₂(O_{1,5}SiCH₂CH₂SiO_{1,5})_{0,25}.

11. Procédé selon l'une quelconque des revendications 1 à 10, où le polyéthylène glycol à terminaison hydroxy a la formule : 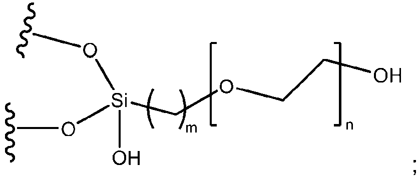 où :
m est un nombre entier d'environ 1 à environ 10 ;
n est un nombre entier d'environ 2 à environ 50 ; et
où les lignes ondulées indiquent les points de fixation à la surface des particules poreuses.

12. Procédé selon la revendication 11, où :
m est égal à 2 ou 3, de préférence où m est égal à 3 et n est d'environ 8 à environ 12 ; et/ou
n est d'environ 5 à environ 15, ou d'environ 8 à environ 12.

13. Procédé selon l'une quelconque des revendications 10 à 12, où les particules poreuses comprennent de la silice, où les particules de silice ont une taille de pore moyenne de 1 000 à environ 2 000 Å, et où au moins une portion de la surface des particules de silice poreuses est modifiée avec un polyéthylène glycol à terminaison méthoxy.

14. Procédé selon la revendication 13, où la portion de la surface modifiée avec le polyéthylène glycol à terminaison méthoxy est le résultat du traitement des particules de silice poreuses avec un réactif polyéthylène glycol à terminaison méthoxy ayant une formule : 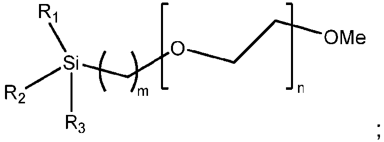 où :
au moins un parmi R₁, R₂ et R₃ est OMe, OEt, CI ou N(CH₃)₂ ;
m est un nombre entier d'environ 1 à environ 10 ; et
n est un nombre entier d'environ 3 à environ 20.

15. Procédé pour réduire une interaction secondaire dans la chromatographie d'exclusion stérique, le procédé comprenant :
a. la fourniture d'un échantillon incluant au moins un analyte ;
b. la fourniture d'un dispositif de chromatographie sur colonne configuré pour détecter la présence ou l'absence d'au moins un analyte dans un échantillon, le dispositif de chromatographie sur colonne comprenant une colonne ayant un intérieur pour recevoir une phase stationnaire, et une phase stationnaire immobilisée au sein de l'intérieur de la colonne, où la phase stationnaire immobilisée comprend des particules poreuses ayant une surface et un diamètre avec une distribution de taille moyenne d'entre environ 1 et environ 20 µm ; une taille de pore moyenne d'environ 40 à environ 2 000 A ; et où lesdites particules poreuses sont modifiées en surface avec un polyéthylène glycol à terminaison hydroxy à une concentration en surface d'environ 0,5 à environ 5,0 µmoles/m² ;
c. la fourniture d'une phase mobile comprenant de l'eau ; un tampon ; et un acide aminé ou un dérivé de celui-ci, où l'acide aminé ou le dérivé de celui-ci est présent dans la phase mobile à une concentration d'environ 5 à environ 40 mM ;
d. l'injection de l'échantillon sur la phase stationnaire immobilisée ;
e. la mise en écoulement de la phase mobile à travers la phase stationnaire immobilisée pendant une période de temps ;
f. l'élution de l'au moins un analyte à partir de la phase stationnaire immobilisée dans la phase mobile ; et
g. la détection de la présence de l'au moins un analyte dans l'échantillon, où un pic dans un chromatogramme indique la présence de l'au moins un analyte dans l'échantillon, et où la réduction de l'interaction secondaire est **caractérisée par** une amélioration d'un ou plusieurs éléments parmi une forme du pic, une aire du pic, une traînée du pic, une récupération de l'analyte ou une diminution de la variabilité inter-essais.
